# EUROPEAN PATENT APPLICATION

(11) **EP 2 363 465 A1**
(43) Date of publication of application: **07.09.2011**
(21) Application number: 09820628.7
(22) Date of filing: 16.10.2009
(51) Int. Cl.: C12N 15/09, A01H 1/00, A01H 5/00

(54) **TRANSGENIC PLANT OF WHICH SEED HAS ENLARGED SIZE**

(30) Priority: 16.10.2008 JP 2008267877
(71) Applicant: Riken, Wako-shi, Saitama 351-0198 (JP); National Institute Of Agrobiological Sciences, Ibaraki 305-8602 (JP)
(72) Inventor: YOSHIZUMI Takeshi, Yokohama-shi Kanagawa 230-0045 (JP); MATSUI Minami, Yokohama-shi Kanagawa 230-0045 (JP); KURIYAMA Tomoko, Yokohama-shi Kanagawa 230-0045 (JP); KURODA Hirofumi, Yokohama-shi Kanagawa 230-0045 (JP); TAKAHASHI Shinya, Yokohama-shi Kanagawa 230-0045 (JP); HIROCHIKA Hirohiko, Tsukuba-shi Ibaraki 305-8602 (JP)
(74) Representative: Vossius & Partner
(86) International application number: PCT/JP2009/067875
(87) International publication number: WO 2010/044450

(57) **Abstract**

The present invention relates to a transgenic plant having increased seed size prepared via introduction of DNA encoding a protein consisting of a specific amino acid sequence derived from a plant, such as rice, and a method for producing the same.

SEQUENCE LISTING

<110>  RIKEN
       National Institute of Agrobiological Sciences

<120>  Transgenic plants having enlarged seeds

<130>  PH-4150-PCT

<150>  JP 2008-267877
<151>  2008-10-16

<160>  13

<170>  PatentIn version 3.4

<210>  1
<211>  1657
<212>  DNA
<213>  Oryza sativa


<220>
<221>  CDS
<222>  (69)..(575)

<400>  1
ggtcgtcgac ccttcgtcga gccgccgccg ccgccgccga tccttctcct cccacctcgc      60

ccgcgccg atg gag cag ctc aac gcc aag ctc tac gac aag tac acc gcc      110
         Met Glu Gln Leu Asn Ala Lys Leu Tyr Asp Lys Tyr Thr Ala
         1               5                   10

ctc aag aag cgg aag ctg ctc gac gag ggg ctg gac cag aag cgg gct      158
Leu Lys Lys Arg Lys Leu Leu Asp Glu Gly Leu Asp Gln Lys Arg Ala
15                  20                  25                  30

gcc gac atc aag gag ctc cag caa gcc atg aaa gat tgg gtc gcc gac      206
Ala Asp Ile Lys Glu Leu Gln Gln Ala Met Lys Asp Trp Val Ala Asp
                35                  40                  45

ctc cag ggc gag aac gag cgc ctc att gcc aag ctg acc caa aag gag      254
Leu Gln Gly Glu Asn Glu Arg Leu Ile Ala Lys Leu Thr Gln Lys Glu
            50                  55                  60

cag cag ctt gtg gag gtc cag aca ctt ttg ctc gat gag act agg aag      302
Gln Gln Leu Val Glu Val Gln Thr Leu Leu Leu Asp Glu Thr Arg Lys
        65                  70                  75

aca aag gag ctc aat agt gaa ata ttg aag ctt cag tgt ctc ctg gct      350
Thr Lys Glu Leu Asn Ser Glu Ile Leu Lys Leu Gln Cys Leu Leu Ala
    80                  85                  90

gag aag aat gat gca aac cat ata gct aca ggg tct cct gac aca acc      398
Glu Lys Asn Asp Ala Asn His Ile Ala Thr Gly Ser Pro Asp Thr Thr
95                  100                 105                 110

gct gaa atg att ata gaa aat caa act cca att cca cct gca aag aag      446
Ala Glu Met Ile Ile Glu Asn Gln Thr Pro Ile Pro Pro Ala Lys Lys
                115                 120                 125

aca cca aag tca aag agc agg gag aga aat atc cac ttc att gaa aaa      494
Thr Pro Lys Ser Lys Ser Arg Glu Arg Asn Ile His Phe Ile Glu Lys
            130                 135                 140

gcg act gtt cct tgt aat ggc ttt cag gag gaa gga aga gat ctt gat      542
Ala Thr Val Pro Cys Asn Gly Phe Gln Glu Glu Gly Arg Asp Leu Asp
        145                 150                 155

tcc tgt aga agg cat atg agc atc tca ggt tag tggtaatata cacacatgca    595
Ser Cys Arg Arg His Met Ser Ile Ser Gly
    160                 165

gtagtgaatt gtttgaactt aaaagtatgt tgtttgcagg gagtgcgaca gaagaaagct    655

ccagtacctg tatgtttcat atgctggctg aatcagtagt tgggatgaaa ttttcagtga    715

agaaccaaac agaaggattt tcactttcag tctcccatga agctagtggt atttggaaca    775

tcaaatttta tgctattatt ttagccacag catagtactg gaatatctca tcatagttat    835

tattcatcat gttttacttg tgttcatttc tctttcacat tcatttgatt ttgcatatag    895

ccacctccat gtttgccaag ctagctgagt attcaagcaa acagttatgc aaagaacaat    955

cttcaacctg aaaaatgtcc tttaaaccac cagaagagtt gtgtgaatac atattttaat   1015

gctttaatat ctttgtcaag catgtacaca tctctttgga gcttagccgt ttgtagcaga   1075

acttgtatac cagcatacag tgtagttaca tgttgggtgc agaacctgag gatcatgtca   1135

ccaatgttag aacatttgat ttgtaaagat cataatcaag tgtaaggaac gaattacatt   1195

tctaactgaa ttttttaaac tgttgtaggc tacagtttca ccctgacatg ggtagaccaa   1255

cctggtggcg gtgaatggtc atatcagtac tcgtcgttgg gcaccctgga caggattgct   1315

atgggctgga tgaaggagga cataaaattc agcagggcca tgtgtcctgt attctttcag   1375

cagatatcgc gcatcctcag acaaggctaa gctcaactgg gaagcttagt ttggatttta   1435

gaggtcctga cttttgctag acagtggact gaaactgact tgttttagga gtgacacgag   1495

gctttatgta tcctttcgtt tggcctttgt tgttcctgtt gtgaacataa caattagcaa   1555

gtgattcgag atgttggtcg cctggttgcc gtatgtcttt atgtatagcc cctgatttga   1615

actctgatgg ttctgaaaat aagagcttcg acattgctgt cc                      1657

<210>  2
<211>  168
<212>  PRT
<213>  Oryza sativa

<400>  2

Met Glu Gln Leu Asn Ala Lys Leu Tyr Asp Lys Tyr Thr Ala Leu Lys
1               5                   10                  15

Lys Arg Lys Leu Leu Asp Glu Gly Leu Asp Gln Lys Arg Ala Ala Asp

20                  25                  30

Ile Lys Glu Leu Gln Gln Ala Met Lys Asp Trp Val Ala Asp Leu Gln
        35                  40                  45

Gly Glu Asn Glu Arg Leu Ile Ala Lys Leu Thr Gln Lys Glu Gln Gln
    50                  55                  60

Leu Val Glu Val Gln Thr Leu Leu Leu Asp Glu Thr Arg Lys Thr Lys
65                  70                  75                  80

Glu Leu Asn Ser Glu Ile Leu Lys Leu Gln Cys Leu Leu Ala Glu Lys
                85                  90                  95

Asn Asp Ala Asn His Ile Ala Thr Gly Ser Pro Asp Thr Thr Ala Glu
            100                 105                 110

Met Ile Ile Glu Asn Gln Thr Pro Ile Pro Pro Ala Lys Lys Thr Pro
        115                 120                 125

Lys Ser Lys Ser Arg Glu Arg Asn Ile His Phe Ile Glu Lys Ala Thr
    130                 135                 140

Val Pro Cys Asn Gly Phe Gln Glu Glu Gly Arg Asp Leu Asp Ser Cys
145                 150                 155                 160

Arg Arg His Met Ser Ile Ser Gly
                165

<210>  3
<211>  1048
<212>  DNA
<213>  Oryza sativa

<220>
<221>  CDS
<222>  (76)..(963)

<400>  3
gatacagcct agaacaactg cttgccaaaa aaggaaaaga aaagaaaaca cttagtgcag      60

aaactgggaa ttgca atg gcg gga gca aga tcc gtt ggt tct ctc cca gtg      111
                 Met Ala Gly Ala Arg Ser Val Gly Ser Leu Pro Val
                 1               5                   10

cct aat gtg cag gcg ctt gca gaa att tgc aat gat cca gat gaa cac      159
Pro Asn Val Gln Ala Leu Ala Glu Ile Cys Asn Asp Pro Asp Glu His
        15                  20                  25

ata cct gaa agg tac atc aga cca gag gct agt tct gag gag gtc atc      207

Ile Pro Glu Arg Tyr Ile Arg Pro Glu Ala Ser Ser Glu Glu Val Ile
    30                  35                  40

aac aac tac caa ggt gac atg gcg att ccg atc atc gat ctc aag aaa      255
Asn Asn Tyr Gln Gly Asp Met Ala Ile Pro Ile Ile Asp Leu Lys Lys
45                  50                  55                  60

ttg ctt tgt ccg caa tca tca gaa gag gag tgt gtg aag ctg aga tct      303
Leu Leu Cys Pro Gln Ser Ser Glu Glu Glu Cys Val Lys Leu Arg Ser
                65                  70                  75

gcc tgc cag tat tgg ggg ttc ttt ctg ctc att aac cat gga gtg cca      351
Ala Cys Gln Tyr Trp Gly Phe Phe Leu Leu Ile Asn His Gly Val Pro
            80                  85                  90

gat gaa gtg att gca aac ctg aag aga gac att gtt gat ttc ttt agc      399
Asp Glu Val Ile Ala Asn Leu Lys Arg Asp Ile Val Asp Phe Phe Ser
        95                  100                 105

cag cca ctg gac acc aag aag gag tac aca cag cta ccg aat agc cta      447
Gln Pro Leu Asp Thr Lys Lys Glu Tyr Thr Gln Leu Pro Asn Ser Leu
    110                 115                 120

gaa ggc tat gga cag agc ttt gtt ttc tct gag gac cag aaa ctt gac      495
Glu Gly Tyr Gly Gln Ser Phe Val Phe Ser Glu Asp Gln Lys Leu Asp
125                 130                 135                 140

tgg gca gac atg ctg tat ctt cat gtc cat cct agc gat tca agg gac      543
Trp Ala Asp Met Leu Tyr Leu His Val His Pro Ser Asp Ser Arg Asp
                145                 150                 155

cta agg ttc tgg ccc act tct cca gca tct ttc agg caa tcc att gat      591
Leu Arg Phe Trp Pro Thr Ser Pro Ala Ser Phe Arg Gln Ser Ile Asp
            160                 165                 170

gca tac tca tca gag act aaa agc ctg gca ctc tgc ttg ttt gag ttc      639
Ala Tyr Ser Ser Glu Thr Lys Ser Leu Ala Leu Cys Leu Phe Glu Phe
        175                 180                 185

atg gct aag gct gtg ggc gct aag cca gag tca ctt tta gat tta ttt      687
Met Ala Lys Ala Val Gly Ala Lys Pro Glu Ser Leu Leu Asp Leu Phe
    190                 195                 200

gaa gag cag cct cga ggc tta agg atg gcc tat tac cca cca tgc cgg      735
Glu Glu Gln Pro Arg Gly Leu Arg Met Ala Tyr Tyr Pro Pro Cys Arg
205                 210                 215                 220

caa gct gac aag gtg atg ggc ctt tcg cca cac tct gat gcg ggt ggc      783
Gln Ala Asp Lys Val Met Gly Leu Ser Pro His Ser Asp Ala Gly Gly
                225                 230                 235

ctg aca ctg ctg ctc gag att aac aat gtg cag ggc ctg cag atc aag      831
Leu Thr Leu Leu Leu Glu Ile Asn Asn Val Gln Gly Leu Gln Ile Lys
            240                 245                 250

aaa gat ggc aag tgg ttc tcc ata gat gct cca aat ggt gca ctt att      879
Lys Asp Gly Lys Trp Phe Ser Ile Asp Ala Pro Asn Gly Ala Leu Ile
        255                 260                 265

gct aac att ggc gac aca ctt gag gta atg acc aat aac aaa tta tgc      927
Ala Asn Ile Gly Asp Thr Leu Glu Val Met Thr Asn Asn Lys Leu Cys

270                 275                 280

ttt cac atg tcg aag gac ctt gat gta ttt gga tga gttcaataaa          973
Phe His Met Ser Lys Asp Leu Asp Val Phe Gly
285                 290                 295

agctcctatt atctagaaaa cttgtcaaag caccaaacaa aatacctaag ttcaaattat   1033

ttatattctt gagtc                                                    1048


<210>  4
<211>  295
<212>  PRT
<213>  Oryza sativa

<400>  4

Met Ala Gly Ala Arg Ser Val Gly Ser Leu Pro Val Pro Asn Val Gln
1               5                   10                  15

Ala Leu Ala Glu Ile Cys Asn Asp Pro Asp Glu His Ile Pro Glu Arg
            20                  25                  30

Tyr Ile Arg Pro Glu Ala Ser Ser Glu Glu Val Ile Asn Asn Tyr Gln
        35                  40                  45

Gly Asp Met Ala Ile Pro Ile Ile Asp Leu Lys Lys Leu Leu Cys Pro
    50                  55                  60

Gln Ser Ser Glu Glu Glu Cys Val Lys Leu Arg Ser Ala Cys Gln Tyr
65                  70                  75                  80

Trp Gly Phe Phe Leu Leu Ile Asn His Gly Val Pro Asp Glu Val Ile
                85                  90                  95

Ala Asn Leu Lys Arg Asp Ile Val Asp Phe Phe Ser Gln Pro Leu Asp
            100                 105                 110

Thr Lys Lys Glu Tyr Thr Gln Leu Pro Asn Ser Leu Glu Gly Tyr Gly
        115                 120                 125

Gln Ser Phe Val Phe Ser Glu Asp Gln Lys Leu Asp Trp Ala Asp Met
    130                 135                 140

Leu Tyr Leu His Val His Pro Ser Asp Ser Arg Asp Leu Arg Phe Trp
145                 150                 155                 160

Pro Thr Ser Pro Ala Ser Phe Arg Gln Ser Ile Asp Ala Tyr Ser Ser
                165                 170                 175

Glu Thr Lys Ser Leu Ala Leu Cys Leu Phe Glu Phe Met Ala Lys Ala
            180                 185                 190

Val Gly Ala Lys Pro Glu Ser Leu Leu Asp Leu Phe Glu Glu Gln Pro
        195                 200                 205

Arg Gly Leu Arg Met Ala Tyr Tyr Pro Pro Cys Arg Gln Ala Asp Lys
    210                 215                 220

Val Met Gly Leu Ser Pro His Ser Asp Ala Gly Gly Leu Thr Leu Leu
225                 230                 235                 240

Leu Glu Ile Asn Asn Val Gln Gly Leu Gln Ile Lys Lys Asp Gly Lys
                245                 250                 255

Trp Phe Ser Ile Asp Ala Pro Asn Gly Ala Leu Ile Ala Asn Ile Gly
            260                 265                 270

Asp Thr Leu Glu Val Met Thr Asn Asn Lys Leu Cys Phe His Met Ser
        275                 280                 285

Lys Asp Leu Asp Val Phe Gly
    290                 295

<210>  5
<211>  1335
<212>  DNA
<213>  Oryza sativa

<220>
<221>  CDS
<222>  (219)..(992)

<400>  5
gagcgagccg agccgagagc gcaacaaaag gaaagcaggg aaacctcgcc gtcctctctc      60

tccatccatg ccctagcacg cgccgcgcag cctacgagcg ggagccacac ctcgaggtct     120

tcatctgttg cactcatgat ttagcaccag ctgaagtgac ctctgagact gcgcggtgac     180

gtagcctgtt gctaggataa aaatctcagt agttaagc atg gcg gat tct atg gtg     236
                                          Met Ala Asp Ser Met Val
                                          1               5

tca agt ttc tgg ggg cct gtt act tca aca act gag ctc tgc gag gaa       284
Ser Ser Phe Trp Gly Pro Val Thr Ser Thr Thr Glu Leu Cys Glu Glu
            10                  15                  20

aat tat gca cac tcg tca tat att gca gaa ttc tac aat act gtt tct       332
Asn Tyr Ala His Ser Ser Tyr Ile Ala Glu Phe Tyr Asn Thr Val Ser
        25                  30                  35

aat gtt cca tgt gtt ctt ctg gca ctt gtt gga ctt gtg aat gct ctt      380
Asn Val Pro Cys Val Leu Leu Ala Leu Val Gly Leu Val Asn Ala Leu
    40                  45                  50

cgc caa ggt ttt gag aaa cga ttc agt gtc ctg cac ata tcc aat atg      428
Arg Gln Gly Phe Glu Lys Arg Phe Ser Val Leu His Ile Ser Asn Met
55                  60                  65                  70

ata ctt gct atc ggc agt atg atc ttc cat gcc acc tta cag cac gtt      476
Ile Leu Ala Ile Gly Ser Met Ile Phe His Ala Thr Leu Gln His Val
                75                  80                  85

tta cag cag agc gat gag act cca atg gtg tgg gag att ctc cta tat      524
Leu Gln Gln Ser Asp Glu Thr Pro Met Val Trp Glu Ile Leu Leu Tyr
            90                  95                  100

ctt tat gta ctt tat tca cca gac tgg cat tac cgg agc act atg cct      572
Leu Tyr Val Leu Tyr Ser Pro Asp Trp His Tyr Arg Ser Thr Met Pro
        105                 110                 115

act ttc ctt ttc cta tac ggt gct gct ttt gca gta gtc cat ttc ctg      620
Thr Phe Leu Phe Leu Tyr Gly Ala Ala Phe Ala Val Val His Phe Leu
    120                 125                 130

gtg cga ttc caa gtg gta ttc aag ttg cat tac gtt ggc ctc tgt ctc      668
Val Arg Phe Gln Val Val Phe Lys Leu His Tyr Val Gly Leu Cys Leu
135                 140                 145                 150

cta tgc atc cca cgg atg tac aag tac tac ata caa act aaa gac atg      716
Leu Cys Ile Pro Arg Met Tyr Lys Tyr Tyr Ile Gln Thr Lys Asp Met
                155                 160                 165

gct gcc aag cgt cta gca aag ctg tgg gtt ctt acg ttg agc ttg gcg      764
Ala Ala Lys Arg Leu Ala Lys Leu Trp Val Leu Thr Leu Ser Leu Ala
            170                 175                 180

act ctt tgc tgg cta ttt gat cga atg ttc tgt aag aag ctt tca cat      812
Thr Leu Cys Trp Leu Phe Asp Arg Met Phe Cys Lys Lys Leu Ser His
        185                 190                 195

tgg tac gtc aac cca caa ggg cac gca tgg tgg cat att ctc atg gga      860
Trp Tyr Val Asn Pro Gln Gly His Ala Trp Trp His Ile Leu Met Gly
    200                 205                 210

ttt aac tca tac ttt gca aac aca ttc tta atg ttt tgc cga gct caa      908
Phe Asn Ser Tyr Phe Ala Asn Thr Phe Leu Met Phe Cys Arg Ala Gln
215                 220                 225                 230

cag cgt ggg tgg gag ccc aaa att acc cac ctt ttc ggg ttc ttg cct      956
Gln Arg Gly Trp Glu Pro Lys Ile Thr His Leu Phe Gly Phe Leu Pro
                235                 240                 245

tat gtc aag att cag aaa ccc caa aag agg gaa tga gttctctatt          1002
Tyr Val Lys Ile Gln Lys Pro Gln Lys Arg Glu
            250                 255

tgtcagtgct gttctccagg accttaaagc caacgccctc atgtgcttag acaagaagca   1062

gaggcatttt ttttgtatat gggcttccaa gtgattacgg actacagtgt tatgatgcct   1122

cactagattt tgttaacttt taactaacgc ctgttttgta ccatacttaa ttgttgctca 1182

gagattagtt ggagtgttgt ctcatagttt tggtcattat tttttgtttc tctacatttg 1242

attttccgtt ctaataggac tgaattttat ttgttttttg tttgtcttga atgttgtcga 1302

acagttttag tcaagttact gcttcatatg gcc 1335

<210> 6
<211> 257
<212> PRT
<213> Oryza sativa

<400> 6

Met Ala Asp Ser Met Val Ser Ser Phe Trp Gly Pro Val Thr Ser Thr
1               5                   10                  15

Thr Glu Leu Cys Glu Glu Asn Tyr Ala His Ser Ser Tyr Ile Ala Glu
            20                  25                  30

Phe Tyr Asn Thr Val Ser Asn Val Pro Cys Val Leu Leu Ala Leu Val
        35                  40                  45

Gly Leu Val Asn Ala Leu Arg Gln Gly Phe Glu Lys Arg Phe Ser Val
    50                  55                  60

Leu His Ile Ser Asn Met Ile Leu Ala Ile Gly Ser Met Ile Phe His
65                  70                  75                  80

Ala Thr Leu Gln His Val Leu Gln Gln Ser Asp Glu Thr Pro Met Val
                85                  90                  95

Trp Glu Ile Leu Leu Tyr Leu Tyr Val Leu Tyr Ser Pro Asp Trp His
            100                 105                 110

Tyr Arg Ser Thr Met Pro Thr Phe Leu Phe Leu Tyr Gly Ala Ala Phe
        115                 120                 125

Ala Val Val His Phe Leu Val Arg Phe Gln Val Val Phe Lys Leu His
    130                 135                 140

Tyr Val Gly Leu Cys Leu Leu Cys Ile Pro Arg Met Tyr Lys Tyr Tyr
145                 150                 155                 160

Ile Gln Thr Lys Asp Met Ala Ala Lys Arg Leu Ala Lys Leu Trp Val
                165                 170                 175

Leu Thr Leu Ser Leu Ala Thr Leu Cys Trp Leu Phe Asp Arg Met Phe

180                 185                 190

Cys Lys Lys Leu Ser His Trp Tyr Val Asn Pro Gln Gly His Ala Trp
        195                 200                 205

Trp His Ile Leu Met Gly Phe Asn Ser Tyr Phe Ala Asn Thr Phe Leu
    210                 215                 220

Met Phe Cys Arg Ala Gln Gln Arg Gly Trp Glu Pro Lys Ile Thr His
225                 230                 235                 240

Leu Phe Gly Phe Leu Pro Tyr Val Lys Ile Gln Lys Pro Gln Lys Arg
                245                 250                 255

Glu

<210>  7
<211>  1631
<212>  DNA
<213>  Oryza sativa

<220>
<221>  CDS
<222>  (1045)..(1269)

<400>  7
gttggtctag gctgtaacta ccttttttta tttactttat ttgtttcctg ctctatgtag      60

tgacatgctg tgttatattg ctgaattata ataccatcaa atagtttgga gaaagtactt     120

tattttttct gctggcatgt ttgaacattc acaatactat atcgtgcata tgtacctctc     180

tgttttggtc ttgtcagtta aaaacgaacg gagatttctt gtgctctctt ttttttagta     240

atttctggag cttaggtttc tttagcgttt gaaaggaatg aggaattgat aatgatcgag     300

atgtcgtgtt atcaactttt ctttcttaaa caatatacta gtcatactcc ttttcctgta     360

tttacagtgt caaatctctg tcagaggatg aatggatact tgacccggtt atcattgctg     420

atatatatga gcaacaggta tatatcttca cttccatcaa tacactagaa actatgcaca     480

ttatgatctt attaaaagag cttttgtgta tcaatggcgt tacactttcc actgtattga     540

tcttttaaag agtgtcattt gttgttttct tataaagaat tataggtgat attgttatta     600

gtacatgctg acccgttatt gtagatttca tagttatcat ctactaggct ttttatacaa      660

gttgtctcta tgatctgctc tgcagctctg gcttgtatgg tagtaactga agcaaaaagt     720

cctgacttac aaaacaattg tgggtgtgac tctgttagtt gcagttgctg gctattttca     780

catgaagcta ttttttacta aatatgattg atgtaattat atgtaaaaag gtaaaaattg      840

taacgaactg cttggaatgt ctagtaaaac tatcatagcg tccattctgc gttactttga     900

tgtgcagttg tgtacccatt cctgggtcat gctgaaacac aaattggcgt gatacgcttc     960

attaattggt gattccattt tcagggcctt gatttcattg tcgatggagg tactagaatt    1020

gctgacccgt ctactgtggt tgat atg acg gga agc tac cct acc atc att       1071
                           Met Thr Gly Ser Tyr Pro Thr Ile Ile
                           1               5

cgt cag gga aag gta tgc att ttc att aat gga cta aag tta tcc tac      1119
Arg Gln Gly Lys Val Cys Ile Phe Ile Asn Gly Leu Lys Leu Ser Tyr
10                  15                  20                  25

cat atg act ata cta tca agc aaa agg aaa ttt ctc att cta tcc cct      1167
His Met Thr Ile Leu Ser Ser Lys Arg Lys Phe Leu Ile Leu Ser Pro
                30                  35                  40

gac att tgg caa atg ata tgt act gca ttt aaa cac acc gtt atg tat      1215
Asp Ile Trp Gln Met Ile Cys Thr Ala Phe Lys His Thr Val Met Tyr
            45                  50                  55

tat ctc gta atg ctt atc tat ttc ata ttt gtg acc tat ttc gtt gac      1263
Tyr Leu Val Met Leu Ile Tyr Phe Ile Phe Val Thr Tyr Phe Val Asp
        60                  65                  70

tta tga ttggcataat tagctaatac tggatgcttc aactgcattt tatgcggttt       1319
Leu

gtataaatgc acagaggccc ttatgtcttc cttttcctgc tttagggccc taagctagat    1379

tggatggtag cggaagatga ggaacaagag tcgcgaccaa catttgcgta taaagcagct    1439

tgagtcactc ttcggctgtc ctatcatagg aggttgtaaa cttgtaatta cttcaccaga    1499

gcctggtctt ttgactcctg aatctgctac tctcagctag accagtatat atactacatg    1559

ttgtaaatat gccagggaat gtaaccagga atcgacatag ttcaccagat tattttcagt    1619

tcactgagcc tc                                                        1631

<210>  8
<211>  74
<212>  PRT
<213>  Oryza sativa

<400>  8

Met Thr Gly Ser Tyr Pro Thr Ile Ile Arg Gln Gly Lys Val Cys Ile
1               5                   10                  15

Phe Ile Asn Gly Leu Lys Leu Ser Tyr His Met Thr Ile Leu Ser Ser
            20                  25                  30

Lys Arg Lys Phe Leu Ile Leu Ser Pro Asp Ile Trp Gln Met Ile Cys
        35                  40                  45

Thr Ala Phe Lys His Thr Val Met Tyr Tyr Leu Val Met Leu Ile Tyr
    50                  55                  60

Phe Ile Phe Val Thr Tyr Phe Val Asp Leu
65                  70

<210>  9
<211>  257
<212>  PRT
<213>  Zea mays

<400>  9

Met Ala Asp Ser Met Val Ala Ser Phe Trp Gly Pro Val Thr Ser Thr
1               5                   10                  15

Thr Glu Leu Cys Glu Glu Asn Tyr Ala His Ser Ser Tyr Ile Ala Glu
            20                  25                  30

Phe Tyr Asn Thr Ile Ser Asn Val Pro Cys Val Leu Leu Ala Leu Ile
        35                  40                  45

Gly Leu Val Asn Ala Phe Cys Gln Gly Phe Glu Lys Arg Phe Ser Val
    50                  55                  60

Leu His Ile Ser Asn Met Ile Leu Ala Ile Gly Ser Met Ile Phe His
65                  70                  75                  80

Ala Thr Leu Gln Leu Leu Leu Gln Gln Ser Asp Glu Thr Pro Met Val
                85                  90                  95

Trp Glu Ile Leu Leu Tyr Met Tyr Val Leu Tyr Ser Pro Asp Trp Asn
            100                 105                 110

Tyr Arg Ser Thr Met Pro Thr Phe Leu Phe Leu Tyr Gly Ala Ala Phe
        115                 120                 125

Ala Val Val His Phe Phe Ala Arg Phe Gln Val Val Phe Lys Leu His
    130                 135                 140

Tyr Val Gly Leu Cys Leu Leu Cys Ile Pro Arg Met Tyr Lys Tyr Tyr
145                 150                 155                 160

Ile Gln Thr Lys Asp Val Gly Ala Lys Arg Leu Ala Lys Leu Trp Val
                165                 170                 175

Leu Thr Leu Thr Leu Gly Thr Leu Cys Trp Leu Val Asp Arg Val Phe
            180                 185                 190

Cys Lys Lys Leu Ser His Trp Tyr Val Asn Pro Gln Gly His Ala Trp
        195                 200                 205

Trp His Val Leu Met Gly Leu Asn Ser Tyr Tyr Ala Asn Thr Phe Leu
    210                 215                 220

Met Phe Cys Arg Ala Gln Gln Arg Gly Trp Glu Pro Arg Ile Thr His
225                 230                 235                 240

Leu Leu Gly Phe Leu Pro Tyr Val Lys Val Gln Lys Pro Glu Lys Arg
                245                 250                 255

Glu

<210>  10
<211>  255
<212>  PRT
<213>  Arabidopsis thaliana

<400>  10

Met Ala Asp Gly Ile Ser Ser Phe Trp Gly Pro Val Thr Ser Thr Ile
1               5                   10                  15

Glu Cys Cys Glu Met Asn Tyr Ala Tyr Ser Ser Tyr Ile Ala Glu Phe
            20                  25                  30

Tyr Asn Thr Ile Ser Asn Val Pro Gly Ile Leu Leu Ala Leu Ile Gly
        35                  40                  45

Leu Val Asn Ala Leu Arg Gln Arg Phe Glu Lys Arg Phe Ser Ile Leu
    50                  55                  60

His Ile Ser Asn Met Ile Leu Ala Ile Gly Ser Met Leu Tyr His Ala
65                  70                  75                  80

Thr Leu Gln His Val Gln Gln Gln Ser Asp Glu Thr Pro Met Val Trp
                85                  90                  95

Glu Ile Leu Leu Tyr Met Tyr Ile Leu Tyr Ser Pro Asp Trp His Tyr
            100                 105                 110

Arg Ser Thr Met Pro Thr Phe Leu Phe Leu Tyr Gly Ala Ala Phe Ala

115                 120                 125

Ile Val His Ala Tyr Leu Arg Phe Gly Ile Gly Phe Lys Val His Tyr
    130                 135                 140

Val Ile Leu Cys Leu Leu Cys Ile Pro Arg Met Tyr Lys Tyr Tyr Ile
145                 150                 155                 160

His Thr Glu Asp Thr Ala Ala Lys Arg Ile Ala Lys Trp Tyr Val Ala
                165                 170                 175

Thr Ile Leu Val Gly Ser Ile Cys Trp Phe Cys Asp Arg Val Phe Cys
            180                 185                 190

Lys Thr Ile Ser Gln Trp Pro Val Asn Pro Gln Gly His Ala Leu Trp
        195                 200                 205

His Val Phe Met Ser Phe Asn Ser Tyr Cys Ala Asn Thr Phe Leu Met
    210                 215                 220

Phe Cys Arg Ala Gln Gln Arg Gly Trp Asn Pro Lys Val Lys Tyr Phe
225                 230                 235                 240

Leu Gly Val Leu Pro Tyr Val Lys Ile Glu Lys Pro Lys Thr Gln
                245                 250                 255

<210>  11
<211>  255
<212>  PRT
<213>  Medicago truncatula

<400>  11

Met Ala Glu Thr Ile Ser Ser Phe Trp Gly Pro Val Thr Ser Thr Ile
1               5                   10                  15

Glu Cys Cys Glu Lys Asn Tyr Ala Tyr Ser Ser Tyr Ile Ala Glu Phe
            20                  25                  30

Tyr Asn Thr Ile Ser Asn Ile Pro Thr Ile Leu Leu Ala Phe Ile Gly
        35                  40                  45

Leu Ile Asn Ala Phe Arg Gln Arg Phe Glu Lys Arg Phe Ser Val Leu
    50                  55                  60

His Val Ser Asn Met Thr Leu Ala Phe Gly Ser Met Leu Tyr His Ala
65                  70                  75                  80

Thr Leu Gln His Val Gln Gln Gln Ser Asp Glu Thr Pro Met Val Trp
                85                  90                  95

Glu Ile Leu Leu Tyr Met Tyr Ile Leu Tyr Ser Pro Asp Trp His Tyr
            100                 105                 110

Arg Ser Thr Met Pro Ile Phe Leu Phe Phe Tyr Gly Ala Ala Phe Ala
        115                 120                 125

Val Val His Ser Phe Val Arg Phe Asp Ile Gly Phe Lys Val His Tyr
    130                 135                 140

Val Ile Leu Cys Leu Leu Cys Ile Pro Arg Met Tyr Lys Tyr Tyr Ile
145                 150                 155                 160

Tyr Thr Ala Asp Val Cys Ala Lys Trp Ile Ala Lys Leu Tyr Val Ala
                165                 170                 175

Thr Leu Leu Leu Gly Ser Leu Phe Trp Leu Cys Asp Arg Val Phe Cys
            180                 185                 190

Lys Glu Ile Ser Gln Trp Pro Val Asn Pro Gln Gly His Ala Leu Trp
        195                 200                 205

His Val Phe Met Gly Leu Asn Ser Tyr Phe Ala Asn Thr Phe Leu Met
    210                 215                 220

Phe Cys Arg Ala Gln Gln Arg Gly Trp Ser Pro Lys Phe Val Arg Leu
225                 230                 235                 240

Phe Gly Val Leu Pro Tyr Val Lys Ile Glu Lys Pro Lys Ser Gln
                245                 250                 255

<210>  12
<211>  30
<212>  DNA
<213>  Artificial

<220>
<223>  primer

<400>  12
gtacgtattt ttacaacaat taccaacaac                                        30

<210>  13
<211>  30
<212>  DNA
<213>  Artificial

<220>
<223>  primer

<400>  13
ggattcaatc ttaagaaact ttattgccaa                                        30

SEQUENCE LISTING

<110> RIKEN
      National Institute of Agrobiological Sciences

<120> Transgenic plant having increased seed size

<130> T1820 EP S3

<140> PCT/JP2009/067875
<141> 2009-10-16

<150> JP 2008-267877
<151> 2008-10-16

<160> 13

<170> PatentIn version 3.4

<210> 1
<211> 1657
<212> DNA
<213> Oryza sativa

<220>
<221> CDS
<222> (69)..(575)

<400> 1
ggtcgtcgac ccttcgtcga gccgccgccg ccgccgccga tccttctcct cccacctcgc      60

ccgcgccg atg gag cag ctc aac gcc aag ctc tac gac aag tac acc gcc      110
         Met Glu Gln Leu Asn Ala Lys Leu Tyr Asp Lys Tyr Thr Ala
         1               5                   10

ctc aag aag cgg aag ctg ctc gac gag ggg ctg gac cag aag cgg gct      158
Leu Lys Lys Arg Lys Leu Leu Asp Glu Gly Leu Asp Gln Lys Arg Ala
15                  20                  25                  30

gcc gac atc aag gag ctc cag caa gcc atg aaa gat tgg gtc gcc gac      206
Ala Asp Ile Lys Glu Leu Gln Gln Ala Met Lys Asp Trp Val Ala Asp

                35                  40                  45

ctc cag ggc gag aac gag cgc ctc att gcc aag ctg acc caa aag gag 254
Leu Gln Gly Glu Asn Glu Arg Leu Ile Ala Lys Leu Thr Gln Lys Glu
            50                  55                  60

cag cag ctt gtg gag gtc cag aca ctt ttg ctc gat gag act agg aag 302
Gln Gln Leu Val Glu Val Gln Thr Leu Leu Leu Asp Glu Thr Arg Lys
        65                  70                  75

aca aag gag ctc aat agt gaa ata ttg aag ctt cag tgt ctc ctg gct 350
Thr Lys Glu Leu Asn Ser Glu Ile Leu Lys Leu Gln Cys Leu Leu Ala
    80                  85                  90

gag aag aat gat gca aac cat ata gct aca ggg tct cct gac aca acc 398
Glu Lys Asn Asp Ala Asn His Ile Ala Thr Gly Ser Pro Asp Thr Thr
95                  100                 105                 110

gct gaa atg att ata gaa aat caa act cca att cca cct gca aag aag 446
Ala Glu Met Ile Ile Glu Asn Gln Thr Pro Ile Pro Pro Ala Lys Lys
                115                 120                 125

aca cca aag tca aag agc agg gag aga aat atc cac ttc att gaa aaa 494
Thr Pro Lys Ser Lys Ser Arg Glu Arg Asn Ile His Phe Ile Glu Lys
            130                 135                 140

gcg act gtt cct tgt aat ggc ttt cag gag gaa gga aga gat ctt gat 542
Ala Thr Val Pro Cys Asn Gly Phe Gln Glu Glu Gly Arg Asp Leu Asp
        145                 150                 155

tcc tgt aga agg cat atg agc atc tca ggt tag tggtaatata cacacatgca 595
Ser Cys Arg Arg His Met Ser Ile Ser Gly
    160                 165

gtagtgaatt gtttgaactt aaaagtatgt tgtttgcagg gagtgcgaca gaagaaagct 655

ccagtacctg tatgtttcat atgctggctg aatcagtagt tgggatgaaa ttttcagtga 715

agaaccaaac agaaggattt tcactttcag tctcccatga agctagtggt atttggaaca 775

tcaaatttta tgctattatt ttagccacag catagtactg gaatatctca tcatagttat 835

tattcatcat gttttacttg tgttcatttc tctttcacat tcatttgatt ttgcatatag 895

ccacctccat gtttgccaag ctagctgagt attcaagcaa acagttatgc aaagaacaat 955

cttcaacctg aaaaatgtcc tttaaaccac cagaagagtt gtgtgaatac atattttaat 1015

gctttaatat ctttgtcaag catgtacaca tctctttgga gcttagccgt ttgtagcaga 1075

acttgtatac cagcatacag tgtagttaca tgttgggtgc agaacctgag gatcatgtca 1135

ccaatgttag aacatttgat ttgtaaagat cataatcaag tgtaaggaac gaattacatt 1195

tctaactgaa ttttttaaac tgttgtaggc tacagtttca ccctgacatg ggtagaccaa 1255

cctggtggcg gtgaatggtc atatcagtac tcgtcgttgg gcaccctgga caggattgct 1315

atgggctgga tgaaggagga cataaaattc agcagggcca tgtgtcctgt attctttcag 1375

cagatatcgc gcatcctcag acaaggctaa gctcaactgg gaagcttagt ttggatttta 1435

gaggtcctga cttttgctag acagtggact gaaactgact tgttttagga gtgacacgag 1495

gctttatgta tcctttcgtt tggcctttgt tgttcctgtt gtgaacataa caattagcaa 1555

gtgattcgag atgttggtcg cctggttgcc gtatgtcttt atgtatagcc cctgatttga 1615

actctgatgg ttctgaaaat aagagcttcg acattgctgt cc 1657

<210> 2
<211> 168
<212> PRT
<213> Oryza sativa

<400> 2

Met Glu Gln Leu Asn Ala Lys Leu Tyr Asp Lys Tyr Thr Ala Leu Lys
1               5                   10                  15

Lys Arg Lys Leu Leu Asp Glu Gly Leu Asp Gln Lys Arg Ala Ala Asp
            20                  25                  30

Ile Lys Glu Leu Gln Gln Ala Met Lys Asp Trp Val Ala Asp Leu Gln
        35                  40                  45

Gly Glu Asn Glu Arg Leu Ile Ala Lys Leu Thr Gln Lys Glu Gln Gln
    50                  55                  60

Leu Val Glu Val Gln Thr Leu Leu Leu Asp Glu Thr Arg Lys Thr Lys
65                  70                  75                  80

Glu Leu Asn Ser Glu Ile Leu Lys Leu Gln Cys Leu Leu Ala Glu Lys
                85                  90                  95

Asn Asp Ala Asn His Ile Ala Thr Gly Ser Pro Asp Thr Thr Ala Glu
            100                 105                 110

Met Ile Ile Glu Asn Gln Thr Pro Ile Pro Pro Ala Lys Lys Thr Pro
        115                 120                 125

Lys Ser Lys Ser Arg Glu Arg Asn Ile His Phe Ile Glu Lys Ala Thr
    130                 135                 140

Val Pro Cys Asn Gly Phe Gln Glu Glu Gly Arg Asp Leu Asp Ser Cys
145                 150                 155                 160

Arg Arg His Met Ser Ile Ser Gly
                165

<210>  3
<211>  1048
<212>  DNA
<213>  Oryza sativa

<220>
<221> CDS
<222> (76)..(963)

<400> 3
gatacagcct agaacaactg cttgccaaaa aaggaaaaga aaagaaaaca cttagtgcag 60

aaactgggaa ttgca atg gcg gga gca aga tcc gtt ggt tct ctc cca gtg 111
Met Ala Gly Ala Arg Ser Val Gly Ser Leu Pro Val
1               5                   10

cct aat gtg cag gcg ctt gca gaa att tgc aat gat cca gat gaa cac 159
Pro Asn Val Gln Ala Leu Ala Glu Ile Cys Asn Asp Pro Asp Glu His
        15                  20                  25

ata cct gaa agg tac atc aga cca gag gct agt tct gag gag gtc atc 207
Ile Pro Glu Arg Tyr Ile Arg Pro Glu Ala Ser Ser Glu Glu Val Ile
    30                  35                  40

aac aac tac caa ggt gac atg gcg att ccg atc atc gat ctc aag aaa 255
Asn Asn Tyr Gln Gly Asp Met Ala Ile Pro Ile Ile Asp Leu Lys Lys
45                  50                  55                  60

ttg ctt tgt ccg caa tca tca gaa gag gag tgt gtg aag ctg aga tct 303
Leu Leu Cys Pro Gln Ser Ser Glu Glu Glu Cys Val Lys Leu Arg Ser
                65                  70                  75

gcc tgc cag tat tgg ggg ttc ttt ctg ctc att aac cat gga gtg cca 351
Ala Cys Gln Tyr Trp Gly Phe Phe Leu Leu Ile Asn His Gly Val Pro
            80                  85                  90

gat gaa gtg att gca aac ctg aag aga gac att gtt gat ttc ttt agc 399
Asp Glu Val Ile Ala Asn Leu Lys Arg Asp Ile Val Asp Phe Phe Ser
        95                  100                 105

cag cca ctg gac acc aag aag gag tac aca cag cta ccg aat agc cta 447
Gln Pro Leu Asp Thr Lys Lys Glu Tyr Thr Gln Leu Pro Asn Ser Leu
    110                 115                 120

gaa ggc tat gga cag agc ttt gtt ttc tct gag gac cag aaa ctt gac 495

Glu Gly Tyr Gly Gln Ser Phe Val Phe Ser Glu Asp Gln Lys Leu Asp
125                 130                 135                 140

tgg gca gac atg ctg tat ctt cat gtc cat cct agc gat tca agg gac     543
Trp Ala Asp Met Leu Tyr Leu His Val His Pro Ser Asp Ser Arg Asp
                145                 150                 155

cta agg ttc tgg ccc act tct cca gca tct ttc agg caa tcc att gat     591
Leu Arg Phe Trp Pro Thr Ser Pro Ala Ser Phe Arg Gln Ser Ile Asp
            160                 165                 170

gca tac tca tca gag act aaa agc ctg gca ctc tgc ttg ttt gag ttc     639
Ala Tyr Ser Ser Glu Thr Lys Ser Leu Ala Leu Cys Leu Phe Glu Phe
        175                 180                 185

atg gct aag gct gtg ggc gct aag cca gag tca ctt tta gat tta ttt     687
Met Ala Lys Ala Val Gly Ala Lys Pro Glu Ser Leu Leu Asp Leu Phe
    190                 195                 200

gaa gag cag cct cga ggc tta agg atg gcc tat tac cca cca tgc cgg     735
Glu Glu Gln Pro Arg Gly Leu Arg Met Ala Tyr Tyr Pro Pro Cys Arg
205                 210                 215                 220

caa gct gac aag gtg atg ggc ctt tcg cca cac tct gat gcg ggt ggc     783
Gln Ala Asp Lys Val Met Gly Leu Ser Pro His Ser Asp Ala Gly Gly
                225                 230                 235

ctg aca ctg ctg ctc gag att aac aat gtg cag ggc ctg cag atc aag     831
Leu Thr Leu Leu Leu Glu Ile Asn Asn Val Gln Gly Leu Gln Ile Lys
            240                 245                 250

aaa gat ggc aag tgg ttc tcc ata gat gct cca aat ggt gca ctt att     879
Lys Asp Gly Lys Trp Phe Ser Ile Asp Ala Pro Asn Gly Ala Leu Ile
        255                 260                 265

gct aac att ggc gac aca ctt gag gta atg acc aat aac aaa tta tgc     927
Ala Asn Ile Gly Asp Thr Leu Glu Val Met Thr Asn Asn Lys Leu Cys
    270                 275                 280

ttt cac atg tcg aag gac ctt gat gta ttt gga tga gttcaataaa          973
Phe His Met Ser Lys Asp Leu Asp Val Phe Gly
285                 290                 295

agctcctatt atctagaaaa cttgtcaaag caccaaacaa aatacctaag ttcaaattat 1033

ttatattctt gagtc 1048

<210> 4
<211> 295
<212> PRT
<213> Oryza sativa

<400> 4

Met Ala Gly Ala Arg Ser Val Gly Ser Leu Pro Val Pro Asn Val Gln
1               5                   10                  15

Ala Leu Ala Glu Ile Cys Asn Asp Pro Asp Glu His Ile Pro Glu Arg
            20                  25                  30

Tyr Ile Arg Pro Glu Ala Ser Ser Glu Glu Val Ile Asn Asn Tyr Gln
        35                  40                  45

Gly Asp Met Ala Ile Pro Ile Ile Asp Leu Lys Lys Leu Leu Cys Pro
    50                  55                  60

Gln Ser Ser Glu Glu Glu Cys Val Lys Leu Arg Ser Ala Cys Gln Tyr
65                  70                  75                  80

Trp Gly Phe Phe Leu Leu Ile Asn His Gly Val Pro Asp Glu Val Ile
                85                  90                  95

Ala Asn Leu Lys Arg Asp Ile Val Asp Phe Phe Ser Gln Pro Leu Asp
            100                 105                 110

Thr Lys Lys Glu Tyr Thr Gln Leu Pro Asn Ser Leu Glu Gly Tyr Gly

        115                 120                 125

Gln Ser Phe Val Phe Ser Glu Asp Gln Lys Leu Asp Trp Ala Asp Met
    130                 135                 140

Leu Tyr Leu His Val His Pro Ser Asp Ser Arg Asp Leu Arg Phe Trp
145                 150                 155                 160

Pro Thr Ser Pro Ala Ser Phe Arg Gln Ser Ile Asp Ala Tyr Ser Ser
                165                 170                 175

Glu Thr Lys Ser Leu Ala Leu Cys Leu Phe Glu Phe Met Ala Lys Ala
            180                 185                 190

Val Gly Ala Lys Pro Glu Ser Leu Leu Asp Leu Phe Glu Glu Gln Pro
        195                 200                 205

Arg Gly Leu Arg Met Ala Tyr Tyr Pro Pro Cys Arg Gln Ala Asp Lys
    210                 215                 220

Val Met Gly Leu Ser Pro His Ser Asp Ala Gly Gly Leu Thr Leu Leu
225                 230                 235                 240

Leu Glu Ile Asn Asn Val Gln Gly Leu Gln Ile Lys Lys Asp Gly Lys
                245                 250                 255

Trp Phe Ser Ile Asp Ala Pro Asn Gly Ala Leu Ile Ala Asn Ile Gly
            260                 265                 270

Asp Thr Leu Glu Val Met Thr Asn Asn Lys Leu Cys Phe His Met Ser
        275                 280                 285

Lys Asp Leu Asp Val Phe Gly
    290                 295

<210>  5
<211>  1335
<212>  DNA
<213>  Oryza sativa

<220>
<221>  CDS
<222>  (219)..(992)

<400>  5
gagcgagccg agccgagagc gcaacaaaag gaaagcaggg aaacctcgcc gtcctctctc      60

tccatccatg ccctagcacg cgccgcgcag cctacgagcg ggagccacac ctcgaggtct     120

tcatctgttg cactcatgat ttagcaccag ctgaagtgac ctctgagact gcgcggtgac     180

gtagcctgtt gctaggataa aaatctcagt agttaagc atg gcg gat tct atg gtg     236
                                          Met Ala Asp Ser Met Val
                                          1               5

tca agt ttc tgg ggg cct gtt act tca aca act gag ctc tgc gag gaa       284
Ser Ser Phe Trp Gly Pro Val Thr Ser Thr Thr Glu Leu Cys Glu Glu
            10                  15                  20

aat tat gca cac tcg tca tat att gca gaa ttc tac aat act gtt tct       332
Asn Tyr Ala His Ser Ser Tyr Ile Ala Glu Phe Tyr Asn Thr Val Ser
        25                  30                  35

aat gtt cca tgt gtt ctt ctg gca ctt gtt gga ctt gtg aat gct ctt       380
Asn Val Pro Cys Val Leu Leu Ala Leu Val Gly Leu Val Asn Ala Leu
    40                  45                  50

cgc caa ggt ttt gag aaa cga ttc agt gtc ctg cac ata tcc aat atg       428
Arg Gln Gly Phe Glu Lys Arg Phe Ser Val Leu His Ile Ser Asn Met
55                  60                  65                  70

ata ctt gct atc ggc agt atg atc ttc cat gcc acc tta cag cac gtt     476
Ile Leu Ala Ile Gly Ser Met Ile Phe His Ala Thr Leu Gln His Val
                75                  80                  85

tta cag cag agc gat gag act cca atg gtg tgg gag att ctc cta tat     524
Leu Gln Gln Ser Asp Glu Thr Pro Met Val Trp Glu Ile Leu Leu Tyr
            90                  95                  100

ctt tat gta ctt tat tca cca gac tgg cat tac cgg agc act atg cct     572
Leu Tyr Val Leu Tyr Ser Pro Asp Trp His Tyr Arg Ser Thr Met Pro
        105                 110                 115

act ttc ctt ttc cta tac ggt gct gct ttt gca gta gtc cat ttc ctg     620
Thr Phe Leu Phe Leu Tyr Gly Ala Ala Phe Ala Val Val His Phe Leu
    120                 125                 130

gtg cga ttc caa gtg gta ttc aag ttg cat tac gtt ggc ctc tgt ctc     668
Val Arg Phe Gln Val Val Phe Lys Leu His Tyr Val Gly Leu Cys Leu
135                 140                 145                 150

cta tgc atc cca cgg atg tac aag tac tac ata caa act aaa gac atg     716
Leu Cys Ile Pro Arg Met Tyr Lys Tyr Tyr Ile Gln Thr Lys Asp Met
                155                 160                 165

gct gcc aag cgt cta gca aag ctg tgg gtt ctt acg ttg agc ttg gcg     764
Ala Ala Lys Arg Leu Ala Lys Leu Trp Val Leu Thr Leu Ser Leu Ala
            170                 175                 180

act ctt tgc tgg cta ttt gat cga atg ttc tgt aag aag ctt tca cat     812
Thr Leu Cys Trp Leu Phe Asp Arg Met Phe Cys Lys Lys Leu Ser His
        185                 190                 195

tgg tac gtc aac cca caa ggg cac gca tgg tgg cat att ctc atg gga     860
Trp Tyr Val Asn Pro Gln Gly His Ala Trp Trp His Ile Leu Met Gly
    200                 205                 210

ttt aac tca tac ttt gca aac aca ttc tta atg ttt tgc cga gct caa     908
Phe Asn Ser Tyr Phe Ala Asn Thr Phe Leu Met Phe Cys Arg Ala Gln
215                 220                 225                 230

cag cgt ggg tgg gag ccc aaa att acc cac ctt ttc ggg ttc ttg cct     956
Gln Arg Gly Trp Glu Pro Lys Ile Thr His Leu Phe Gly Phe Leu Pro

                235                 240                 245

tat gtc aag att cag aaa ccc caa aag agg gaa tga gttctctatt 1002
Tyr Val Lys Ile Gln Lys Pro Gln Lys Arg Glu
            250                 255

tgtcagtgct gttctccagg accttaaagc caacgccctc atgtgcttag acaagaagca 1062

gaggcatttt ttttgtatat gggcttccaa gtgattacgg actacagtgt tatgatgcct 1122

cactagattt tgttaacttt taactaacgc ctgttttgta ccatacttaa ttgttgctca 1182

gagattagtt ggagtgttgt ctcatagttt tggtcattat tttttgtttc tctacatttg 1242

attttccgtt ctaataggac tgaattttat ttgttttttg tttgtcttga atgttgtcga 1302

acagttttag tcaagttact gcttcatatg gcc 1335

<210> 6
<211> 257
<212> PRT
<213> Oryza sativa

<400> 6

Met Ala Asp Ser Met Val Ser Ser Phe Trp Gly Pro Val Thr Ser Thr
1               5                   10                  15

Thr Glu Leu Cys Glu Glu Asn Tyr Ala His Ser Ser Tyr Ile Ala Glu
            20                  25                  30

Phe Tyr Asn Thr Val Ser Asn Val Pro Cys Val Leu Leu Ala Leu Val
        35                  40                  45

Gly Leu Val Asn Ala Leu Arg Gln Gly Phe Glu Lys Arg Phe Ser Val
    50                  55                  60

Leu His Ile Ser Asn Met Ile Leu Ala Ile Gly Ser Met Ile Phe His
65                  70                  75                  80

Ala Thr Leu Gln His Val Leu Gln Gln Ser Asp Glu Thr Pro Met Val
                85                  90                  95

Trp Glu Ile Leu Leu Tyr Leu Tyr Val Leu Tyr Ser Pro Asp Trp His
            100                 105                 110

Tyr Arg Ser Thr Met Pro Thr Phe Leu Phe Leu Tyr Gly Ala Ala Phe
        115                 120                 125

Ala Val Val His Phe Leu Val Arg Phe Gln Val Val Phe Lys Leu His
    130                 135                 140

Tyr Val Gly Leu Cys Leu Leu Cys Ile Pro Arg Met Tyr Lys Tyr Tyr
145                 150                 155                 160

Ile Gln Thr Lys Asp Met Ala Ala Lys Arg Leu Ala Lys Leu Trp Val
                165                 170                 175

Leu Thr Leu Ser Leu Ala Thr Leu Cys Trp Leu Phe Asp Arg Met Phe
            180                 185                 190

Cys Lys Lys Leu Ser His Trp Tyr Val Asn Pro Gln Gly His Ala Trp
        195                 200                 205

Trp His Ile Leu Met Gly Phe Asn Ser Tyr Phe Ala Asn Thr Phe Leu
    210                 215                 220

Met Phe Cys Arg Ala Gln Gln Arg Gly Trp Glu Pro Lys Ile Thr His
225                 230                 235                 240

Leu Phe Gly Phe Leu Pro Tyr Val Lys Ile Gln Lys Pro Gln Lys Arg
                245                 250                 255

Glu

<210>  7
<211>  1631
<212>  DNA
<213>  Oryza sativa

<220>
<221>  CDS
<222>  (1045)..(1269)

<400>  7
gttggtctag gctgtaacta ccttttttta tttactttat ttgtttcctg ctctatgtag      60

tgacatgctg tgttatattg ctgaattata ataccatcaa atagtttgga gaaagtactt     120

tattttttct gctggcatgt ttgaacattc acaatactat atcgtgcata tgtacctctc     180

tgttttggtc ttgtcagtta aaaacgaacg gagatttctt gtgctctctt ttttttagta     240

atttctggag cttaggtttc tttagcgttt gaaaggaatg aggaattgat aatgatcgag     300

atgtcgtgtt atcaactttt ctttcttaaa caatatacta gtcatactcc ttttcctgta     360

tttacagtgt caaatctctg tcagaggatg aatggatact tgacccggtt atcattgctg     420

atatatatga gcaacaggta tatatcttca cttccatcaa tacactagaa actatgcaca     480

ttatgatctt attaaaagag cttttgtgta tcaatggcgt tacactttcc actgtattga     540

tcttttaaag agtgtcattt gttgttttct tataaagaat tataggtgat attgttatta     600

gtacatgctg acccgttatt gtagatttca tagttatcat ctactaggct ttttatacaa      660

gttgtctcta tgatctgctc tgcagctctg gcttgtatgg tagtaactga agcaaaaagt 720

cctgacttac aaaacaattg tgggtgtgac tctgttagtt gcagttgctg gctattttca 780

catgaagcta ttttttacta aatatgattg atgtaattat atgtaaaaag gtaaaaattg 840

taacgaactg cttggaatgt ctagtaaaac tatcatagcg tccattctgc gttactttga 900

tgtgcagttg tgtacccatt cctgggtcat gctgaaacac aaattggcgt gatacgcttc 960

attaattggt gattccattt tcagggcctt gatttcattg tcgatggagg tactagaatt 1020

gctgacccgt ctactgtggt tgat atg acg gga agc tac cct acc atc att 1071
Met Thr Gly Ser Tyr Pro Thr Ile Ile
1               5

cgt cag gga aag gta tgc att ttc att aat gga cta aag tta tcc tac 1119
Arg Gln Gly Lys Val Cys Ile Phe Ile Asn Gly Leu Lys Leu Ser Tyr
10                  15                  20                  25

cat atg act ata cta tca agc aaa agg aaa ttt ctc att cta tcc cct 1167
His Met Thr Ile Leu Ser Ser Lys Arg Lys Phe Leu Ile Leu Ser Pro
                30                  35                  40

gac att tgg caa atg ata tgt act gca ttt aaa cac acc gtt atg tat 1215
Asp Ile Trp Gln Met Ile Cys Thr Ala Phe Lys His Thr Val Met Tyr
            45                  50                  55

tat ctc gta atg ctt atc tat ttc ata ttt gtg acc tat ttc gtt gac 1263
Tyr Leu Val Met Leu Ile Tyr Phe Ile Phe Val Thr Tyr Phe Val Asp
        60                  65                  70

tta tga ttggcataat tagctaatac tggatgcttc aactgcattt tatgcggttt 1319
Leu

gtataaatgc acagaggccc ttatgtcttc cttttcctgc tttagggccc taagctagat 1379

tggatggtag cggaagatga ggaacaagag tcgcgaccaa catttgcgta taaagcagct 1439

tgagtcactc ttcggctgtc ctatcatagg aggttgtaaa cttgtaatta cttcaccaga 1499

gcctggtctt ttgactcctg aatctgctac tctcagctag accagtatat atactacatg 1559

ttgtaaatat gccagggaat gtaaccagga atcgacatag ttcaccagat tattttcagt 1619

tcactgagcc tc 1631

<210> 8
<211> 74
<212> PRT
<213> Oryza sativa

<400> 8

Met Thr Gly Ser Tyr Pro Thr Ile Ile Arg Gln Gly Lys Val Cys Ile
1               5                   10                  15

Phe Ile Asn Gly Leu Lys Leu Ser Tyr His Met Thr Ile Leu Ser Ser
            20                  25                  30

Lys Arg Lys Phe Leu Ile Leu Ser Pro Asp Ile Trp Gln Met Ile Cys
        35                  40                  45

Thr Ala Phe Lys His Thr Val Met Tyr Tyr Leu Val Met Leu Ile Tyr
    50                  55                  60

Phe Ile Phe Val Thr Tyr Phe Val Asp Leu
65                  70

<210> 9
<211> 257
<212> PRT
<213> Zea mays

<400> 9

Met Ala Asp Ser Met Val Ala Ser Phe Trp Gly Pro Val Thr Ser Thr
1               5                   10                  15

Thr Glu Leu Cys Glu Glu Asn Tyr Ala His Ser Ser Tyr Ile Ala Glu
            20                  25                  30

Phe Tyr Asn Thr Ile Ser Asn Val Pro Cys Val Leu Leu Ala Leu Ile
        35                  40                  45

Gly Leu Val Asn Ala Phe Cys Gln Gly Phe Glu Lys Arg Phe Ser Val
    50                  55                  60

Leu His Ile Ser Asn Met Ile Leu Ala Ile Gly Ser Met Ile Phe His
65                  70                  75                  80

Ala Thr Leu Gln Leu Leu Leu Gln Gln Ser Asp Glu Thr Pro Met Val
                85                  90                  95

Trp Glu Ile Leu Leu Tyr Met Tyr Val Leu Tyr Ser Pro Asp Trp Asn
            100                 105                 110

Tyr Arg Ser Thr Met Pro Thr Phe Leu Phe Leu Tyr Gly Ala Ala Phe
        115                 120                 125

Ala Val Val His Phe Phe Ala Arg Phe Gln Val Val Phe Lys Leu His
    130                 135                 140

Tyr Val Gly Leu Cys Leu Leu Cys Ile Pro Arg Met Tyr Lys Tyr Tyr
145                 150                 155                 160

Ile Gln Thr Lys Asp Val Gly Ala Lys Arg Leu Ala Lys Leu Trp Val

                165                 170                 175

Leu Thr Leu Thr Leu Gly Thr Leu Cys Trp Leu Val Asp Arg Val Phe
            180                 185                 190

Cys Lys Lys Leu Ser His Trp Tyr Val Asn Pro Gln Gly His Ala Trp
        195                 200                 205

Trp His Val Leu Met Gly Leu Asn Ser Tyr Tyr Ala Asn Thr Phe Leu
    210                 215                 220

Met Phe Cys Arg Ala Gln Gln Arg Gly Trp Glu Pro Arg Ile Thr His
225                 230                 235                 240

Leu Leu Gly Phe Leu Pro Tyr Val Lys Val Gln Lys Pro Glu Lys Arg
                245                 250                 255

Glu

<210> 10
<211> 255
<212> PRT
<213> Arabidopsis thaliana

<400> 10

Met Ala Asp Gly Ile Ser Ser Phe Trp Gly Pro Val Thr Ser Thr Ile
1               5                   10                  15

Glu Cys Cys Glu Met Asn Tyr Ala Tyr Ser Ser Tyr Ile Ala Glu Phe
            20                  25                  30

Tyr Asn Thr Ile Ser Asn Val Pro Gly Ile Leu Leu Ala Leu Ile Gly
        35                  40                  45

Leu Val Asn Ala Leu Arg Gln Arg Phe Glu Lys Arg Phe Ser Ile Leu
    50                  55                  60

His Ile Ser Asn Met Ile Leu Ala Ile Gly Ser Met Leu Tyr His Ala
65                  70                  75                  80

Thr Leu Gln His Val Gln Gln Gln Ser Asp Glu Thr Pro Met Val Trp
                85                  90                  95

Glu Ile Leu Leu Tyr Met Tyr Ile Leu Tyr Ser Pro Asp Trp His Tyr
            100                 105                 110

Arg Ser Thr Met Pro Thr Phe Leu Phe Leu Tyr Gly Ala Ala Phe Ala
        115                 120                 125

Ile Val His Ala Tyr Leu Arg Phe Gly Ile Gly Phe Lys Val His Tyr
    130                 135                 140

Val Ile Leu Cys Leu Leu Cys Ile Pro Arg Met Tyr Lys Tyr Tyr Ile
145                 150                 155                 160

His Thr Glu Asp Thr Ala Ala Lys Arg Ile Ala Lys Trp Tyr Val Ala
                165                 170                 175

Thr Ile Leu Val Gly Ser Ile Cys Trp Phe Cys Asp Arg Val Phe Cys
            180                 185                 190

Lys Thr Ile Ser Gln Trp Pro Val Asn Pro Gln Gly His Ala Leu Trp
        195                 200                 205

His Val Phe Met Ser Phe Asn Ser Tyr Cys Ala Asn Thr Phe Leu Met
    210                 215                 220

Phe Cys Arg Ala Gln Gln Arg Gly Trp Asn Pro Lys Val Lys Tyr Phe
225                 230                 235                 240

Leu Gly Val Leu Pro Tyr Val Lys Ile Glu Lys Pro Lys Thr Gln
                245                 250                 255

<210>  11
<211>  255
<212>  PRT
<213>  Medicago truncatula

<400>  11

Met Ala Glu Thr Ile Ser Ser Phe Trp Gly Pro Val Thr Ser Thr Ile
1               5                   10                  15

Glu Cys Cys Glu Lys Asn Tyr Ala Tyr Ser Ser Tyr Ile Ala Glu Phe
            20                  25                  30

Tyr Asn Thr Ile Ser Asn Ile Pro Thr Ile Leu Leu Ala Phe Ile Gly
        35                  40                  45

Leu Ile Asn Ala Phe Arg Gln Arg Phe Glu Lys Arg Phe Ser Val Leu
    50                  55                  60

His Val Ser Asn Met Thr Leu Ala Phe Gly Ser Met Leu Tyr His Ala
65                  70                  75                  80

Thr Leu Gln His Val Gln Gln Gln Ser Asp Glu Thr Pro Met Val Trp

                85                  90                  95

Glu Ile Leu Leu Tyr Met Tyr Ile Leu Tyr Ser Pro Asp Trp His Tyr
            100                 105                 110

Arg Ser Thr Met Pro Ile Phe Leu Phe Phe Tyr Gly Ala Ala Phe Ala
        115                 120                 125

Val Val His Ser Phe Val Arg Phe Asp Ile Gly Phe Lys Val His Tyr
    130                 135                 140

Val Ile Leu Cys Leu Leu Cys Ile Pro Arg Met Tyr Lys Tyr Tyr Ile
145                 150                 155                 160

Tyr Thr Ala Asp Val Cys Ala Lys Trp Ile Ala Lys Leu Tyr Val Ala
                165                 170                 175

Thr Leu Leu Leu Gly Ser Leu Phe Trp Leu Cys Asp Arg Val Phe Cys
            180                 185                 190

Lys Glu Ile Ser Gln Trp Pro Val Asn Pro Gln Gly His Ala Leu Trp
        195                 200                 205

His Val Phe Met Gly Leu Asn Ser Tyr Phe Ala Asn Thr Phe Leu Met
    210                 215                 220

Phe Cys Arg Ala Gln Gln Arg Gly Trp Ser Pro Lys Phe Val Arg Leu
225                 230                 235                 240

Phe Gly Val Leu Pro Tyr Val Lys Ile Glu Lys Pro Lys Ser Gln
                245                 250                 255

<210> 12
<211> 30
<212> DNA
<213> Artificial

<220>
<223> primer

<400> 12
gtacgtattt ttacaacaat taccaacaac 30

<210> 13
<211> 30
<212> DNA
<213> Artificial

<220>
<223> primer

<400> 13
ggattcaatc ttaagaaact ttattgccaa 30

## Description

### Technical Field

The present invention relates to a transgenic plant having increased seed size and a method for producing the same.

### Background Art

In order to increase plant seed size, cross breeding and random mutagenesis of genes of interest have heretofore been carried out. However, no satisfactory mutagenesis techniques of target genes of plants have yet been established. Thus, artificial modification has been difficult. Although seed size-related genes were isolated, most of such genes were identified as genes regulating seed size because seed sizes were increased or decreased when such genes were destroyed.

An example of a transgenic plant into which a gene increasing seed size has been introduced is a transgenic plant having increased seed weight resulting from introduction of the rice-derived PLASTOCHRON1 gene (Accession Number of the National Center for Biotechnology Information (NCBI): AB096259) with the use of a binary vector via electroporation into a rice plant (JP Patent Publication (kokai) No. 2005-204621 A). Such transgenic plant was prepared by introducing a rice-derived gene into a rice plant. When such gene is introduced into a plant other than rice, however, whether or not seed size could be increased remains unknown.

### Disclosure of the Invention

### Objects to Be Attained by the Invention

The objects of the present invention are to identify DNA related to increased seed size and to provide a transgenic plant having seed size that is increased via introduction of such DNA into a plant and a method for producing the same.

### Means for Attaining the Objects

The present inventors have conducted concentrated studies in order to attain the above objects. As a result, they identified a gene related to increased seed size with the utilization of the Fox hunting system, introduced such gene into a plant, and allowed such gene to overexpress therein, thereby completing the present invention.

The present invention is summarized as follows.
[1] A transgenic plant into which DNA encoding any of proteins (a) to (c) below has been introduced so as to be capable of being expressed therein:
   (a) a protein comprising the amino acid sequence as shown in SEQ ID NO: 2, 4, 6, or 8;
   (b) a protein comprising an amino acid sequence derived from the amino acid sequence as shown in SEQ ID NO: 2, 4, 6, or 8 by deletion, substitution, or addition of one or several amino acids and having activity of increasing seed size; or
   (c) a protein comprising an amino acid sequence having 90% or higher identity with the amino acid sequence as shown in SEQ ID NO: 2, 4, 6, or 8 and having activity of increasing seed size.
[2] A transgenic plant into which any of DNAs (d) to (g) below has been introduced so as to be capable of being expressed therein:
   (d) DNA comprising the nucleotide sequence as shown in SEQ ID NO: 1, 3, 5, or 7;
   (e) DNA comprising a nucleotide sequence derived from the nucleotide sequence as shown in SEQ ID NO: 1, 3, 5, or 7 by deletion, substitution, or addition of one or several nucleotides and encoding a protein having activity of increasing seed size;
   (f) DNA comprising a nucleotide sequence having 90% or higher identity with the nucleotide sequence as shown in SEQ ID NO: 1, 3, 5, or 7 and encoding a protein having activity of increasing seed size; or
   (g) DNA hybridizing, under stringent conditions, to DNA comprising a nucleotide sequence complementary to DNA consisting of the nucleotide sequence as shown in SEQ ID NO: 1, 3, 5, or 7 and encoding a protein having activity of increasing seed size.
[3] A transgenic plant into which DNA encoding any of proteins (h) to (j) below has been introduced so as to be capable of being expressed therein:
   (h) a protein comprising the amino acid sequence as shown in SEQ ID NO: 9, 10, or 11;
   (i) a protein comprising an amino acid sequence derived from the amino acid sequence as shown in SEQ ID NO: 9, 10, or 11 by deletion, substitution, or addition of one or several amino acids and having activity of increasing seed size; or
   (j) a protein comprising an amino acid sequence having 90% or higher identity with the amino acid sequence as shown in SEQ ID NO: 9, 10, or 11 and having activity of increasing seed size.
[4] The transgenic plant according to any of [1] to [3], wherein the activity of increasing seed size is activity of increasing surface area, volume, or weight of seed.
[5] The transgenic plant according to any of [1] to [4], which is a plant, part of a plant, cultured plant cell, or seed.
[6] A recombinant vector comprising DNA encoding any of proteins (a) to (c) below:
   (a) a protein comprising the amino acid sequence as shown in SEQ ID NO: 2, 4, 6, or 8;
   (b) a protein comprising an amino acid sequence derived from the amino acid sequence as shown in SEQ ID NO: 2, 4, 6, or 8 by deletion, substitution, or addition of one or several amino acids and having activity of increasing seed size; or
   (c) a protein comprising an amino acid sequence having 90% or higher identity with the amino acid sequence as shown in SEQ ID NO: 2, 4, 6, or 8 and having activity of increasing seed size.
[7] A recombinant vector comprising any of DNAs (d) to (g) below:
   (d) DNA comprising the nucleotide sequence as shown in SEQ ID NO: 1, 3, 5, or 7;
   (e) DNA comprising a nucleotide sequence derived from the nucleotide sequence as shown in SEQ ID NO: 1, 3, 5, or 7 by deletion, substitution, or addition of one or several nucleotides and encoding a protein having activity of increasing seed size;
   (f) DNA comprising a nucleotide sequence having 90% or higher identity with the nucleotide sequence as shown in SEQ ID NO: 1, 3, 5, or 7 and encoding a protein having activity of increasing seed size; or
   (g) DNA hybridizing, under stringent conditions, to DNA comprising a nucleotide sequence complementary to DNA consisting of the nucleotide sequence as shown in SEQ ID NO: 1, 3, 5, or 7 and encoding a protein having activity of increasing seed size.
[8] A recombinant vector comprising DNA encoding any of proteins (h) to (j) below:
   (h) a protein comprising the amino acid sequence as shown in SEQ ID NO: 9, 10, or 11;
   (i) a protein comprising an amino acid sequence derived from the amino acid sequence as shown in SEQ ID NO: 9, 10, or 11 by deletion, substitution, or addition of one or several amino acids and having activity of increasing seed size; or
   (j) a protein comprising an amino acid sequence having 90% or higher identity with the amino acid sequence as shown in SEQ ID NO: 9, 10, or 11 and having activity of increasing seed size.
[9] A method for producing a transgenic plant comprising introducing DNA encoding any of proteins (a) to (c) below into a plant cell and cultivating a plant:
   (a) a protein comprising the amino acid sequence as shown in SEQ ID NO: 2, 4, 6, or 8;
   (b) a protein comprising an amino acid sequence derived from the amino acid sequence as shown in SEQ ID NO: 2, 4, 6, or 8 by deletion, substitution, or addition of one or several amino acids and having activity of increasing seed size; or
   (c) a protein comprising an amino acid sequence having 90% or higher identity with the amino acid sequence as shown in SEQ ID NO: 2, 4, 6, or 8 and having activity of increasing seed size.
[10] A method for producing a transgenic plant comprising introducing any of DNAs (d) to (g) below into a plant cell and cultivating a plant:
   (d) DNA comprising the nucleotide sequence as shown in SEQ ID NO: 1, 3, 5, or 7;
   (e) DNA comprising a nucleotide sequence derived from the nucleotide sequence as shown in SEQ ID NO: 1, 3, 5, or 7 by deletion, substitution, or addition of one or several nucleotides and encoding a protein having activity of increasing seed size;
   (f) DNA comprising a nucleotide sequence having 90% or higher identity with the nucleotide sequence as shown in SEQ ID NO: 1, 3, 5, or 7 and encoding a protein having activity of increasing seed size; or
   (g) DNA hybridizing, under stringent conditions, to DNA comprising a nucleotide sequence complementary to DNA consisting of the nucleotide sequence as shown in SEQ ID NO: 1, 3, 5, or 7 and encoding a protein having activity of increasing seed size.
[11] A method for producing a transgenic plant comprising introducing DNA encoding any of proteins (h) to (j) below into a plant cell and cultivating a plant:
   (h) a protein comprising the amino acid sequence as shown in SEQ ID NO: 9, 10, or 11;
   (i) a protein comprising an amino acid sequence derived from the amino acid sequence as shown in SEQ ID NO: 9, 10, or 11 by deletion, substitution, or addition of one or several amino acids and having activity of increasing seed size; or
   (j) a protein comprising an amino acid sequence having 90% or higher identity with the amino acid sequence as shown in SEQ ID NO: 9, 10, or 11 and having activity of increasing seed size.
[12] The method according to any of [9] to [11], wherein DNA is introduced with the use of the recombinant vector according to any of [6] to [8].

The contents as disclosed in the description and/or drawings of Japanese Patent Application No. 2008-267877, to which the present application claims priority, are incorporated herein.

### Effects of the Invention

DNA used in the present invention is related to increased seed size, and introduction of such DNA into a plant and overexpression therein result in the production of a plant having increased seed size. Since such DNA can be expressed at a high level in a plant if a transformation technique has been established for the plant, the present invention is applicable to any plant. DNA used in the present invention is capable of increasing seed size even if it is introduced into a plant belonging to a different family. Also, the transgenic plant of the present invention enables the production of crops exhibiting higher seed production. By increasing seed size, the commodity value of a plant can be enhanced.

### Brief Description of the Drawings

Fig. 1 shows a scheme of the examples.
Fig. 2 shows wild-type seeds and T2 seeds of K06835 and K15507.
Fig. 3 shows wild-type seeds and T2 seeds of K32722.
Fig. 4 shows wild-type seeds and T2 seeds of K42340.
Fig. 5 shows the area of wild-type seeds and the area of T2 generation seeds of K32722 and K42340.
Fig. 6 shows histograms showing the area of wild-type seeds and T2 generation seeds of K32722.
Fig. 7 shows histograms showing the area of wild-type seeds and T2 generation seeds of K42340.
Fig. 8 shows the volumes of wild-type seeds and T2 generation seeds of K32722.
Fig. 9 shows a comparison of amino acid sequences (corn, *Arabidopsis thaliana,* and alfalfa) of proteins having high identity (i.e., homologous proteins) with the amino acid sequence encoded by DNA (AK073303) introduced into K32722.

### Best Mode for Carrying Out the Invention

### (1) DNA related to increased seed size

DNA related to increased seed size used in the present invention encodes any of proteins (a) to (c) below:
(a) a protein comprising the amino acid sequence as shown in SEQ ID NO: 2, 4, 6, or 8;
(b) a protein comprising an amino acid sequence derived from the amino acid sequence as shown in SEQ ID NO: 2, 4, 6, or 8 by deletion, substitution, or addition of one or several amino acids and having activity of increasing seed size; or
(c) a protein comprising an amino acid sequence having 90% or higher identity with the amino acid sequence as shown in SEQ ID NO: 2, 4, 6, or 8 and having activity of increasing seed size.

Alternatively, DNA related to increased seed size used in the present invention is any of DNAs (d) to (g) below:
(d) DNA comprising the nucleotide sequence as shown in SEQ ID NO: 1, 3, 5, or 7;
(e) DNA comprising a nucleotide sequence derived from the nucleotide sequence as shown in SEQ ID NO: 1, 3, 5, or 7 by deletion, substitution, or addition of one or several nucleotides and encoding a protein having activity of increasing seed size;
(f) DNA comprising a nucleotide sequence having 90% or higher identity with the nucleotide sequence as shown in SEQ ID NO: 1, 3, 5, or 7 and encoding a protein having activity of increasing seed size; or
(g) DNA hybridizing, under stringent conditions, to DNA comprising a nucleotide sequence complementary to DNA consisting of the nucleotide sequence as shown in SEQ ID NO: 1, 3, 5, or 7 and encoding a protein having activity of increasing seed size.

Alternatively, DNA related to increased seed size used in the present invention encodes any of proteins (h) to (j) below:
(h) a protein comprising the amino acid sequence as shown in SEQ ID NO: 9, 10, or 11;
(i) a protein comprising an amino acid sequence derived from the amino acid sequence as shown in SEQ ID NO: 9, 10, or 11 by deletion, substitution, or addition of one or several amino acids and having activity of increasing seed size; or
(j) a protein comprising an amino acid sequence having 90% or higher identity with the amino acid sequence as shown in SEQ ID NO: 9, 10, or 11 and having activity of increasing seed size.

The term "protein (or DNA) having activity of increasing seed size" used herein refers to a protein (or DNA) that is directly or indirectly related to an increase in seed size.

The term "DNA" used herein encompasses genomic DNA, a gene, cDNA, and the like.

The term "activity of increasing seed size" used herein refers to any activity, provided that such activity makes seed size larger than that of wild-type seeds. Examples thereof include activity of increasing surface area, volumes, or weight of seed. Seed size is not particularly limited, provided that seeds are larger than wild-type seeds at statistically significant levels. For example, it is preferable that the seeds be larger than wild-type seeds by at least 5%, 10%, 15%, 20%, 25%, 30%, 35%, 40%, 45%, or 50%.

The term "an amino acid sequence derived from the amino acid sequence by deletion, substitution, or addition of one or several amino acids" used herein means, for example, that 1 to 10 and preferably 1 to 5 amino acids may be deleted in a given amino acid sequence, that 1 to 10 and preferably 1 to 5 amino acids may be substituted with other amino acids in a given amino acid sequence, or that 1 to 10 and preferably 1 to 5 amino acids may be added to a given amino acid sequence.

The term "identity" used in the expression "an amino acid sequence having 90% or higher identity with the amino acid sequence" herein refers to identity of 90% or higher, preferably 95% or higher, more preferably 98% or higher, and further preferably 99% or higher.

The term "identity" used herein refers to the degree of matching between two amino acid sequences (or nucleotide sequences) aligned so as to maximize the number of identical amino acid residues (or the number of identical nucleotides). Specifically, the identity is represented by the percentage (%) of the number of identical amino acid residues (or the number of identical nucleotides) relative to the total number of amino acid residues (or the total number of nucleotides). The percentage identity can be determined with the use of known algorithms, such as BLAST or FASTA. When gaps are introduced as in the case of FASTA, the number of gaps is included in the total number of amino acid residues (or the total number of nucleotides).

The term "a nucleotide sequence derived from the nucleotide sequence by deletion, substitution, or addition of one or several nucleotides" used herein means, for example, that 1 to 10 and preferably 1 to 5 nucleotides may be deleted in a given nucleotide sequence, that 1 to 10 and preferably 1 to 5 nucleotides may be substituted with other nucleotides in a given nucleotide sequence, or that 1 to 10 and preferably 1 to 5 nucleotides may be added to a given nucleotide sequence.

The term "identity" used in the expression "a nucleotide sequence having 90% or higher identity with the nucleotide sequence" herein refers to identity of 90% or higher, preferably 95% or higher, more preferably 98% or higher, and further preferably 99% or higher.

The term "stringent conditions" used herein refers to conditions under which so-called specific hybrids are formed whereas substantially no non-specific hybrids are formed. Under such stringent conditions, for example, a complementary strand of a nucleic acid having high identity (i.e., DNA consisting of a nucleotide sequence having identity of 90% or higher, preferably 95% or higher, more preferably 98% or higher, and further preferably 99% or higher to a given nucleotide sequence) undergoes hybridization whereas a complementary strand of a nucleic acid having identity lower than such level does not undergo hybridization. More specifically, the sodium salt concentration is 15 to 750 mM, preferably 50 to 750 mM, and more preferably 300 to 750 mM, temperature is 25°C to 70°C, preferably 50°C to 70°C, and more preferably 55°C to 65°C, and the formamide concentration is 0% to 50%, preferably 20% to 50%, and more preferably 35% to 45%. Under stringent conditions, further, a filter that had been subjected to hybridization is then washed at a sodium salt concentration of generally 15 to 600 mM, preferably 50 to 600 mM, and more preferably 300 to 600 mM and temperature of 50°C to 70°C, preferably 55°C to 70°C, and more preferably 60°C to 65°C.

Under stringent conditions, alternatively, hybridization may be carried out in the presence of 2x to 6× sodium chloride/sodium citrate (SSC, 1× SSC is 150 mM sodium chloride and 15 mM sodium citrate; pH 7.0) at room temperature to 40°C, and washing may then be carried out in the presence of 0.1x to 1× SSC (preferably 0.1x to 0.2× SSC) and 0.1% SDS at 50°C to 68°C once or a plurality of times.

DNA used in the present invention can be obtained in the form of a nucleic acid fragment via PCR amplification with the use of primers designed based on a DNA sequence encoding the amino acid sequence as shown in SEQ ID NO: 2, 4, 6, 8, 9, 10, or 11 or a DNA sequence as shown in SEQ ID NO: 1, 3, 5, or 7 and a nucleic acid obtained from cDNA library or genomic DNA library as a template. Such DNA can be obtained in the form of a nucleic acid fragment via hybridization using a nucleic acid obtained from the above library or the like as a template and a DNA fragment, which is part of such DNA, as a probe. Alternatively, such DNA may be synthesized in the form of a nucleic acid fragment via various techniques of nucleic acid synthesis known in the art, such as chemical synthesis.

Deletion, substitution, or addition of DNA or amino acid as described above can be carried out by modifying DNA encoding a relevant protein by a technique known in the art. Mutation can be introduced into DNA via, for example, the Kunkel method or the Gapped duplex method. Mutation can be introduced with the use of a mutagenesis kit utilizing site-directed mutagenesis (e.g., Mutant-K (Takara Bio Inc.) or the LA PCR in vitro mutagenesis kit (Takara Bio Inc.)) (Sambrook et al., 1989, Molecular Cloning: Laboratory Manual, 2nd edition, Cold Spring Harbor Laboratory Press; Ausubel et al., 1995, Short Protocols in Molecular Biology, third edition, John Wiley & Sons, Inc.).

DNA used in the present invention was found to have activity of increasing seed size for the first time by measuring the sizes of seeds obtained from a transgenic line with the utilization of the Fox hunting system and identifying cDNA that had been introduced into a line producing seeds significantly larger than wild-type seeds.

The Fox hunting system (i.e.. the full-length cDNA over-expressor gene hunting system) is a technique for elucidating gene fuctions based on changes in traits resulting from introduction and high expression of full-length cDNA in a plant (JP Patent Publication (Saikohyo) 2003-018808 A). In the present invention, rice full-length cDNA is used as full-length cDNA to be introduced into a plant, and an example of a plant into which full-length cDNA can be introduced is, but is not limited to, *Arabidopsis thaliana.*

Specifically, DNA used in the present invention can be identified with the utilization of the Fox hunting system in the following manner.

Specifically, approximately 13,000 independent rice full-length cDNAs are prepared as a pool having equivalent ratios of the cDNAs (referred to as "normalization"), and the cDNAs are integrated into a T-DNA vector having a regulatory region, such as a promoter, an enhancer (e.g., a transcription enhancer E21 or an omega sequence), and a terminator, and a selection marker such as a drug-resistance gene. The resulting T-DNA vectors are introduced into *Agrobacterium,* a rice full-length cDNA expression library (referred to as a rice FOX library) is produced, and whether or not such library reflects the entire cDNA distribution is then confirmed. Thereafter, *Arabidopsis thaliana* is transformed using the *Agrobacterium* as described above by the floral dipping method to produce transgenic *Arabidopsis thaliana* lines (rice FOX lines). In the FOX hunting system, only one or two clone(s) is/are introduced per plant even when plants are infected with a library consisting of hundreds of millions of clones. Thus, transgenic lines in which a different clone (or clones) is/are introduced for each plant can be produced.

T1 seeds are collected from the primary recombinant *Arabidopsis thaliana* (T0) of the approximately 20,000 thus-obtained rice FOX lines, sown on a medium containing antibiotics, and selected. Thus, T1-generation transformants are obtained. The seeds obtained via self-pollination of such transformants are designated as T2 seeds. The T2 seeds (for example, 50 grains per line) are visually observed, and, simultaneously, analyzed with the use of a seed morphology measurement program WINSEEDLE (Regent Instruments) by importing an image showing the seeds as the data into a computer. WINSEEDLE is a program that measures morphology, size, and color of seeds based on images of seeds. This program measures the lengths and the widths of seeds that have been imported into a computer, and the areas thereof are then determined. Wild-type *Arabidopsis thaliana* seeds are used as controls, and lines from which seeds that are significantly larger than wild-type controls are predominantly produced are selected. The seeds of the selected lines are sown and cultivated, and it is confirmed whether or not a phenotype exhibiting increased seed size has been reproduced. Genomic DNAs are extracted from the seeds that had reproduced the phenotype of interest, and the introduced rice full-length cDNAs are isolated via PCR. Such cDNAs are designated as candidates for DNAs causing increased seed size. The candidate DNAs are introduced into *Arabidopsis thaliana* again and overexpressed therein, whether or not seed size is increased is confirmed, and the confirmed rice full-length cDNAs are determined to be DNAs having activity of increasing seed size (i.e., DNAs encoding proteins having activity of increasing seed size).

DNAs identified in such a manner are registered with NCBI under the accession numbers indicated below:
AK100982 (DNA sequence: SEQ ID NO: 1; amino acid sequence: SEQ ID NO: 2);
AK099678 (DNA sequence: SEQ ID NO:3; amino acid sequence: SEQ ID NO: 4);
AK073303 (DNA sequence: SEQ ID NO:5; amino acid sequence: SEQ ID NO: 6);
AK071204 (DNA sequence: SEQ ID NO:7; amino acid sequence: SEQ ID NO: 8).

The above amino acid sequences derived from rice are compared with amino acid sequences derived from other plant species, and DNA encoding a highly identical amino acid sequence and encoding a protein having activity of increasing seed size can be used in the present invention. The identity can be inspected by, for example, accessing sequence databases of NCBI or EMBL (The European Molecular Biology Laboratory) with the use of a sequence homology search program such as BLAST or FASTA (e.g., Alteschul, S. F. et al., 1990, J. Mol. Biol. 15: 403-410; Karlin, S. and Altschul S. F., 1990, Proc. Natl. Acad. Sci., U.S.A., 87: 2264-2268).

Examples of amino acid sequences derived from other plant species having high identity with the amino acid sequence of AK073303 include a corn-derived amino acid sequence (SEQ ID NO: 9; NCBI Accession Numbers: ACF86069 (the amino acid sequence) and BT041064 (the DNA sequence)), an *Arabidopsis thaliana*-derived amino acid sequence (SEQ ID NO: 10; NCBI Accession Numbers: NP#567660 (the amino acid sequence) and NM#118359 (the DNA sequence)), and an alfalfa (*Medicago truncatula*)-derived amino acid sequence (SEQ ID NO: 11; NCBI Accession Numbers: ABD28483 (the amino acid sequence) and AC148915.2 (the DNA sequence)). (2) Recombinant vector

The recombinant vector of the present invention used for plant transformation can be constructed by introducing DNA encoding any of proteins (a) to (c) and (h) to (j) above or any of DNAs (d) to (g) above into an appropriate vector. As the vector, pBI-based, pPZP-based, pSMA-based, and other vectors, which can introduce target DNA into a plant via *Agrobacterium,* are preferably used. In particular, a pBI-based binary vector or intermediate vector is preferably used, and examples thereof include pBI121, pBI101, pBI101.2, pBI101.3, and pBIG2113 vectors. A binary vector is a shuttle vector that is replicable in *Escherichia coli* and *Agrobacterium.* When a plant is infected with *Agrobacterium* containing a binary vector, DNA held between border sequences consisting of an LB sequence and an RB sequence on the vector can be integrated into the nuclear DNA of the plant. Examples of other vectors include a pUC-based vector that can directly introduce DNA into a plant, such as pUC18, pUC19, and pUC9 vectors. Further examples of other vectors include plant virus vectors, such as cauliflower mosaic virus (CaMV), bean golden mosaic virus (BGMV), and tobacco mosaic virus (TMV) vectors.

When a binary vector-based plasmid is used, target DNA is inserted between the border sequences (between LB and RB) of the aforementioned binary vector, and the recombinant vector is multiplied in *E*. *coli.* Subsequently, the multiplied recombinant vectors are introduced into, for example, *Agrobacterium tumefaciens* GV3101, C58, LBA4404, EHA101, or EHA105 or *Agrobacterium rhizogenes* LBA1334 via electroporation or other means, and the target DNA is introduced into a plant using such *Agrobacterium.*

In order to insert target DNA into a vector, at the outset, a method in which purified DNA is cleaved with an appropriate restriction enzyme and the resulting DNA is then ligated to a vector by inserting it into a restriction enzyme site or a multicloning site of an appropriate vector DNA is employed, for example.

Also, it is necessary to integrate target DNA into a vector in a manner that allows the DNA to exert its function. In this regard, a promoter, an enhancer, a terminator, an origin of replication required when using a binary vector system (such as an origin of replication derived from a Ti or Ri plasmid), a selection marker gene, and the like can be ligated to the vector at a region upstream, inside, or downstream of the target DNA.

The promoter is not necessarily derived from a plant, provided that it is DNA that can function in a plant cell and induce expression in a specific tissue or at a specific developmental stage of a plant. Specific examples thereof include a cauliflower mosaic virus (CaMV) 35S promoter, a promoter of the nopaline-synthase gene, an ubiquitin promoter derived from corn, an actin promoter derived from rice, and a PR protein promoter derived from tobacco.

Examples of the enhancers include an enhancer region containing an upstream sequence of the CaMV 35S promoter, a transcription enhancer E12, and an omega sequence, and such enhancers are used to improve expression efficiency of target DNA.

The terminator may be any sequence that can terminate gene transcription driven by the promoter, and examples thereof include a terminator of the nopaline-synthase (NOS) gene, a terminator, of the octopine-synthase (OCS) gene, and a terminator of the CaMV 35S RNA gene.

Examples of the selection marker genes include a hygromycin resistance gene, an ampicillin resistance gene, a neomycin resistance gene, a bialaphos resistance gene, and a dihydrofolate reductase gene.

### (3) Transgenic plant and method for producing the same

The transgenic plant of the present invention can be produced by introducing DNA encoding any of proteins (a) to (c) and (h) to (j) above, any of DNAs (d) to (g) above, or the aforementioned recombinant vector into a target plant. In the present invention, "introduction of DNA" means a situation in which target DNA is introduced into the aforementioned host plant cell by, for example, a known genetic engineering method so as to be capable of being expressed therein. The DNA thus introduced can be integrated into genomic DNA of a host plant, or it can remain contained within an exogenous vector.

The condition "so as to be capable of being expressed" used herein is a condition under which DNA can be constitutively or inductively expressed in the presence of a regulatory sequence, such as a promoter or enhancer.

Introduction of DNA or a recombinant vector may be carried out by a known method, such as an *Agrobacterium* method, a PEG-calcium phosphate method, electroporation, a liposome method, a particle gun method, or a microinjection method. The *Agrobacterium* method includes a method using a protoplast, a method using a piece of tissue, and a method using a plant body (the in planta method). The method using a protoplast can be carried out as a method in which a protoplast is co-cultured with *Agrobacterium* containing a Ti plasmid or an Ri plasmid (*Agrobacterum tumefaciens* or *Agrobacterium rhizogenes,* respectively) and a method in which a protoplast is fused with a spheroplast of *Agrobacterium* (a spheroplast method). The method using a piece of tissue can be carried out via, for example, infection of sterile cultured leaf discs of a target plant or infection of calluses (undifferentiated cultured cells). Also, the in planta method using a seed or a plant body can be carried out by directly treating an imbibed seed, a young plant (a young seedling), a potted plant, and the like with *Agrobacterium.* Such plant transformation methods can be carried out according to the description in, for example, "Shinpan Model shokubutsu no jikken protocol, Idengakutekishuho kara genome kaiseki made" (literal translation: "New edition, Experimental protocol for model plant, From genetic technique to genome analysis") (Supervised by Ko Shimamoto and Kiyotaka Okada, Shujunsha Co., Ltd., 2001).

Whether or not DNA is integrated into the plant body can be confirmed via, for example, PCR, Southern hybridization, or Northern hybridization. For example, DNA is prepared from the transgenic plant, primers specific to the target DNA are designed, and PCR is carried out. Subsequently, the amplified product is subjected to agarose electrophoresis, polyacrylamide gel electrophoresis, or capillary electrophoresis and then stained with ethidium bromide, an SYBR Green solution, or the like, so that the amplified product is detected as a single band. Thus, transformation can be confirmed. Also, PCR can be carried out using a primer that has been labeled with a fluorescent dye or the like in advance, and the amplified product can then be detected. Further, transformation may also be confirmed by a method in which the amplified product is bound to a solid phase such as a microplate, following which the amplified product is detected by a fluorescence, enzymatic, or other reaction.

Alternatively, transformation can also be confirmed by producing a vector having one of various reporter genes, such as a gene for β-glucuronidase (GUS), luciferase (LUC), green fluorescent protein (GFP), chloramphenicol acetyltransferase (CAT), or β-galactosidase (LacZ) ligated to a region downstream of the target DNA, transforming a plant using *Agrobacterium* into which the vector was introduced as described above, and then measuring the expression level of the reporter gene.

Plants to be used for transformation in the present invention can be either monocotyledons or dicotyledons, and examples thereof include, but are not limited to, plants belonging to the family *Brassicaceae* (including *Arabidopsis thaliana,* cabbage, and rapeseed), the family *Leguminosae* (including garden pea, soybean, alfalfa, *Phaseolus angularis,* horse bean, and *Vigna sinensis*), the family *Poaceae* (including rice, corn, barley, and wheat), the family *Compositae* (including safflower and sunflower), the family *Pedaliaceae* (e.g., sesame), and the family *Euphorbiaceae* (including *Jatropha curcas* and *Ricinus communis*). Seed plants, such as edible seed plants and seed plants for oil extraction, are particularly preferable.

In the present invention, a plant material to be subjected to transformation may be any plant organ or tissue, such as a stem, a leaf, a seed, an embryo, an ovule, an ovary, a shoot apex, an anther, and pollen, sections of the above plant organs and tissues, an undifferentiated callus, and a cultured plant cell such as a protoplast obtained by removing the cell wall of the callus by enzymatic treatment. When the in planta method is employed, an imbibed seed and an entire plant body can also be used.

When a cultured plant cell is subjected to transformation, an organ or an organism may be regenerated by a known tissue culture method in order to regenerate a transformant from the resulting transformed cell. A person skilled in the art can easily perform the procedure as described above using a generally known method for regenerating a plant body from a plant cell. For example, regeneration of a plant body from a plant cell can be performed as follows.

When a plant tissue or protoplast is used as a plant material to be subjected to transformation, the plant tissue or protoplast is cultured in a medium for callus formation, which is prepared by adding inorganic elements, vitamins, carbon sources, sugars as energy sources, plant growth regulators (plant hormones, such as auxin, cytokinin, gibberellin, abscisic acid, ethylene, and brassinosteroid), and the like, followed by sterilization. The plant tissue or protoplast is then allowed to form a dedifferentiated callus, which will proliferate into an amorphous mass (hereinafter referred to as "callus induction"). The callus thus formed is transferred to a fresh medium containing plant growth regulators, such as auxin, and is subjected to further proliferation (subculture).

When callus induction is performed on a solid medium such as agar and subculture is performed via liquid culture, for example, each culture can be efficiently carried out in a large amount. Subsequently, callus that has proliferated via subculture is cultured under appropriate conditions to induce re-differentiation of an organ (hereinafter referred to as "re-differentiation induction"), and a complete plant body is regenerated in the end. The re-differentiation induction can be carried out by appropriately setting the types and the amounts of constituents in the medium, such as plant growth regulators such as auxin, as well as carbon sources, light, temperature, and other conditions. An adventitious embryo, an adventitious root, an adventitious shoot, an adventitious stem, an adventitious leaf, and the like are formed, and these adventitious organs further grow into a complete plant body via re-differentiation induction. Alternatively, such adventitious organs can be stored in the form before they grow into a complete plant body (in the form of an encapsulated artificial seed, dried embryo, or lyophilized cell or tissue, for example).

The transgenic plant of the present invention encompasses any of an entire plant body, a part of a plant body (e.g., a leaf, a petal, a stem, a root, and pollen), a cultured plant cell (e.g., a callus and a protoplast), and a seed into which DNA encoding any of proteins (a) to (c) and (h) to (j) above or any of DNAs (d) to (g) above have been introduced. Further, it encompasses progeny plant bodies obtained via sexual or asexual reproduction of plant body and parts, cultured cells, and seeds of the progeny plant body. The transgenic plant of the present invention can be produced in large quantities by obtaining a reproductive material, such as a seed and a protoplast, from the transgenic plant and cultivating or culturing the reproductive material.

### Examples

Hereafter, the present invention is described in greater detail with reference to the following examples, although the present invention is not limited thereto.

### (1) Production of rice FOX line by FOX hunting system

In the present example, pBIG2113SF, which was obtained by introducing an SfiI cloning site into a constitutive expression vector, pBIG2113N (Taji, T. et al., Plant J., 2002, 24 (4): pp. 417-426; and Becker, D. et al., Nucleic Acid Res., 1990, 18 (1): p. 203), was used as a vector below.

### (i) Production of normalized rice full-length cDNA mix

Full-length cDNA was prepared from rice by the CAP trapper method. The resulting cDNA was cloned into a site between SfiI restriction enzyme sites in Lambda ZAP or Lambda pLC-1-B (reference: Seki, M. et al., Plant J., 15, 707-720, 1998). Sequences at the 5' end and the 3' end of cDNA were sequenced using the vector sequence, cDNAs were grouped, and 20,000 independent clones were identified (reference: Seki, M. et al., Plant Physiol. Biochem., 39, 211-220, 2001). Subsequently, a 0.5-µl aliquot was taken from each clone prepared at 50 ng/µl, and all the aliquots were mixed in a single tube. One microliter of the mixture was used to transform 20 µl of the electric competent cell DH10B (Gibco BRL, U.S.A.). Approximately 200,000 independent colonies grown on agar media containing Amp were mixed, and plasmids were collected therefrom. The plasmids thus obtained were designated as the normalized rice full-length cDNA mix.

### (ii) Production of rice FOX library

The normalized rice full-length cDNA mix (2 µg) and 700 µg of pBIG2113SF were mixed together, and completely cleaved with SfiI simultaneously. Thereafter, the cleaved products were concentrated via isopropanol precipitation. The precipitate was dissolved in 8 µl of water, and mixed with 1 µl of 10× buffer and 1 µl of T4 ligase, and a reaction was allowed to proceed overnight at 16°C. The reaction solution (2 µl) was mixed with 40 µl of the Electric competent cell DH10B, and transformation was performed.

Approximately 150,000 independent colonies grown on agar media containing kanamycin (Km) were mixed, and plasmids were collected therefrom. The plasmid solution thus collected (2 µl) was mixed with 40 µl of the Electric competent Agrobacterium cell GV3101, and transformation was performed. Approximately 150,000 independent colonies grown on agar media containing Km were suspended in LB liquid medium, glycerol was added thereto to prepare a 15% glycerol solution, and the resulting solution was stored at -80°C. The glycerol solution thus obtained was designated as a rice FOX library.

### (iii) Production of rice FOX line

Approximately 200,000 colonies of the aforementioned rice FOX library were grown and suspended in a dipping solution, and floral dipping of wild-type *Arabidopsis thaliana* (Colombia (Col-0)) was performed. Seeds (T1 seeds) were harvested and allowed to germinate on nutrient-poor media, BAM, containing hygromycin, and only approximately 20,000 plant lines exhibiting hygromycin resistance were then transplanted to soil. The plants were allowed to self-pollinate, the resulting seeds were designated as T2 seeds, and such seeds were used for seed size observation.

### (2) Selection of rice FOX line via observation of T2 seeds, identification of nucleotide sequence of causal DNA, and reintroduction of DNA into Arabidopsis thaliana

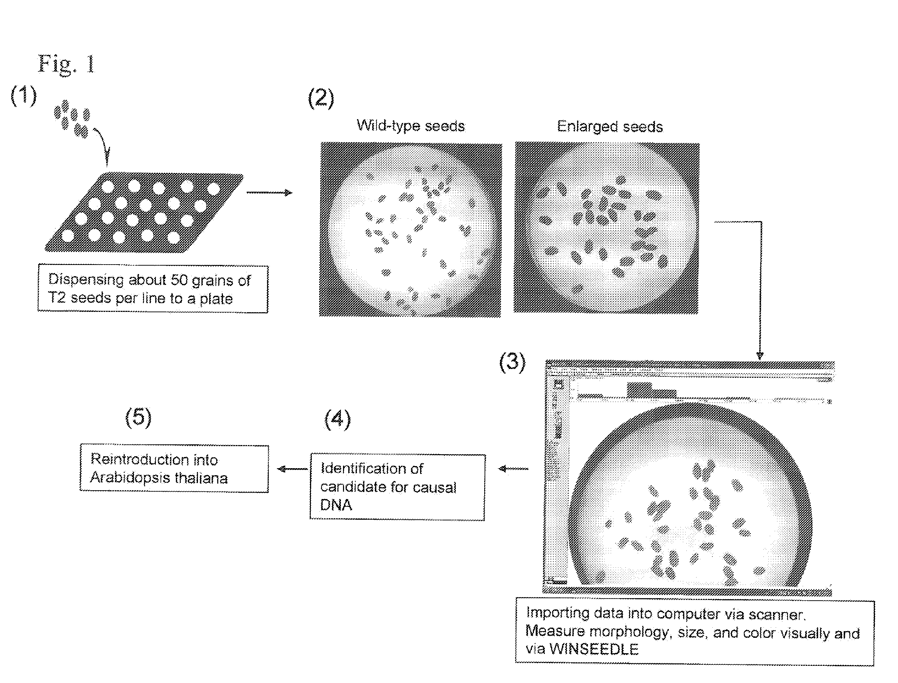

The T2 seeds were placed in wells of a plastic plate in amounts of approximately 50 grains per line (Fig. 1 (1)). While the seeds were visually inspected, an image showing the seeds was imported as the data into a computer (Fig. 1(2)). At this time, lines predominantly producing seeds that were significantly larger than wild-type seeds were selected. The image imported into a computer was analyzed by the WINSEEDLE program by measuring lengths and widths of seeds in the image, and the areas were then determined (Fig. 1(3)). The seeds of the selected lines were sown and cultivated, and it was confirmed whether or not a phenotype exhibiting increased seed size would be reproduced in the subsequent generation. Genomic DNAs were extracted from the seeds that had reproduced the phenotype of interest, the introduced rice full-length cDNAs were isolated via PCR, and the nucleotide sequences were identified.

PCR was carried out using a PCR reaction solution having the composition shown in Table 1, and a cycle of 94°C for 0.5 minutes, 55°C for 0.5 minutes, and 72°C for 4 minutes was repeated 40 times.

**Table 1**

| Composition of the reaction solution: | |
|---|---|
| Primers (100 pM) | 2 × 0.25 µl |
| dNTP (200 µM) | 4 µl |
| Buffer (x2) | 25 µl |
| Polymerase | 0.5 µl |
| Genomic DNA | 10 µl |
| Distilled water | 10 µl |
| Total | 50 µl |

The primers used for PCR were as follows:
GTACGTATTTTTACAACAATTACCAACAAC (SEQ ID NO: 12);
GGATTCAATCTTAAGAAACTTTATTGCCAA (SEQ ID NO: 13).

The PCR products were collected from agarose gels, mixed with pBIG2113SF, completely cleaved with SfiI, precipitated by isopropanol, and then treated with T4 ligase. The resultants were then used to transform *E*. *coli.* Plasmids into which the PCR fragments had been inserted were selected, and the nucleotide sequences of the cDNA fragments of the inserted candidates for causal DNAs were identified using the aforementioned primers (Fig. 1(4)).

The candidates for causal DNAs were overexpressed again in *Arabidopsis thaliana* in the same manner as that used for the FOX hunting system (Fig. 1(5)), and whether seed size would be increased was inspected. The confirmed rice full-length cDNA was designated as DNA that increases seed size.

### (3) DNA that increases seed size

DNAs that were found to increase seed size in (2) above were those introduced into the four rice FOX lines indicated by the numbers below. The numbers in parentheses indicate the NCBI Accession Numbers and the SEQ ID NOs of the nucleotide sequences used herein of the DNAs introduced into the rice FOX lines.
K06835 (AK100982, SEQ ID NO: 1);
K15507 (AK099678, SEQ ID NO: 3);
K32722 (AK073303, SEQ ID NO: 5);
K42340 (AK071204, SEQ ID NO: 7).

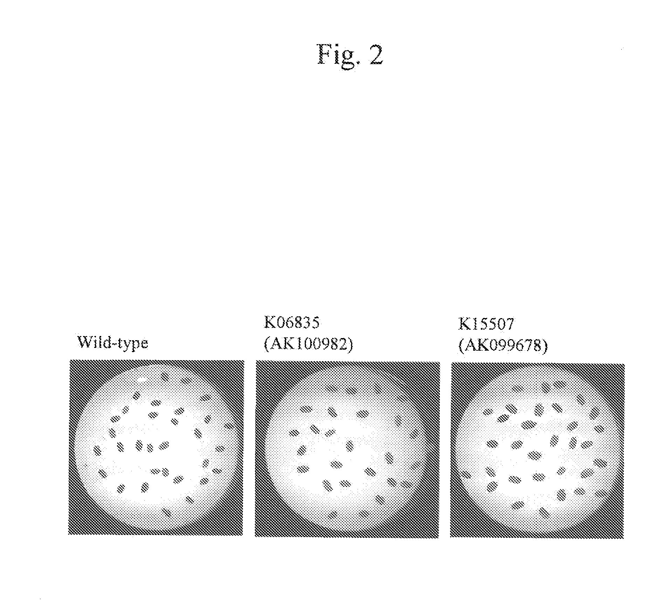

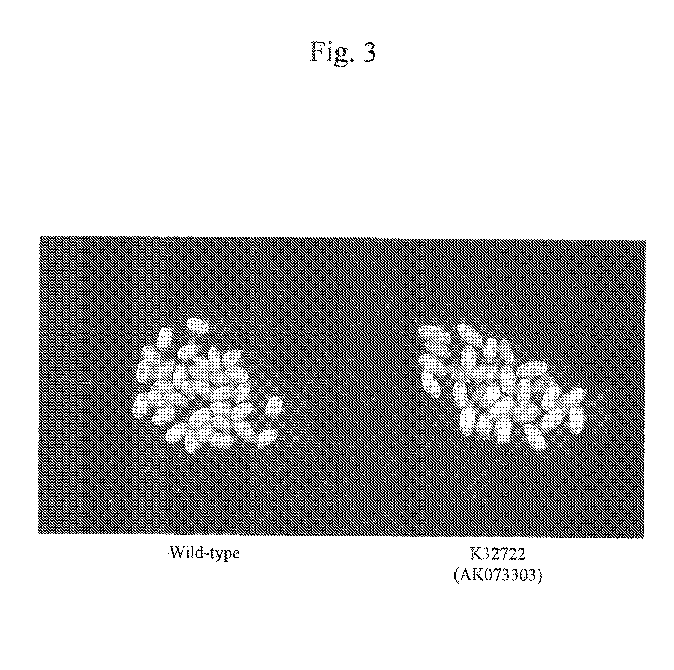

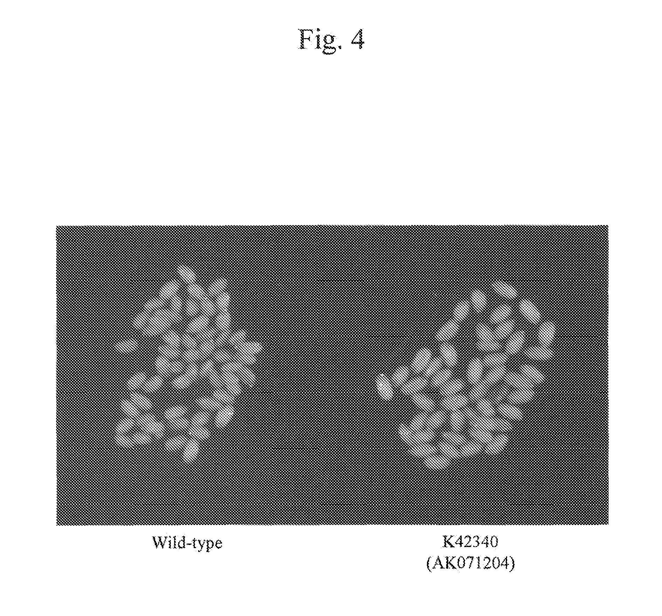

Fig. 2 shows wild-type seeds and T2 seeds of K06835 and K15507. Fig. 3 shows wild-type seeds and T2 seeds of K32722. Fig. 4 shows wild-type seeds and T2 seeds of K42340. Based on the figures, the seeds of the rice FOX lines were found to be larger than wild-type seeds.

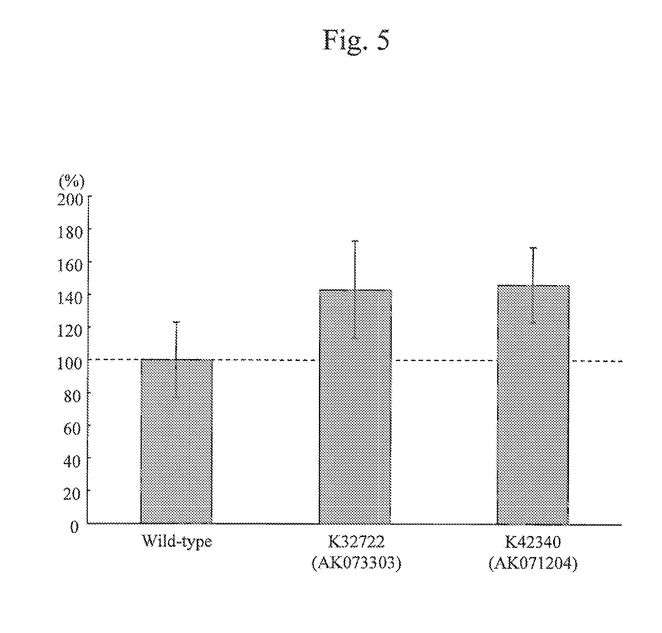

The areas of wild-type seeds were compared with the areas of the seeds (T2 generation seeds) obtained by reintroducing DNAs that had been introduced into K32722 and K42340 in *Arabidopsis thaliana* and overexpressing the same therein (Fig. 5). Compared with wild-type seeds, the areas of K32722 and K42340 seeds were found to have increased by approximately 40%.

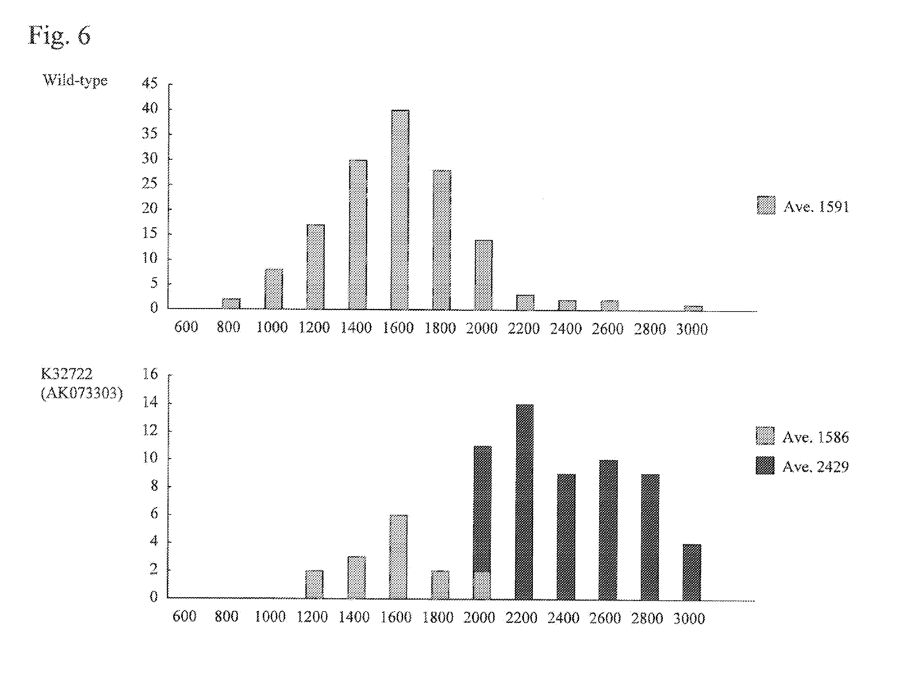

Fig. 6 shows histograms showing the areas of wild-type seeds and T2 generation seeds of K32722 (i.e., distribution of seed areas of K32722-overexpressing lines). The vertical axis indicates the seed number and the horizontal axis indicates the areas (i.e., the pixel numbers). Wild-type seeds exhibited a large peak at a pixel number of 1,600, and such peak has a symmetric normal distribution-like shape. In contrast, the K32722 seeds exhibited a large peak and a small peak. The small peak (gray) appeared at the same position as that for the wild-type seeds (i.e., at a pixel number of 1,600), and the large peak (black) appeared at a position representing areas larger than those of wild-type seeds (i.e., at a pixel number of 2,200). Since the phenotype of the transformant appeared predominantly in the segregating generation, the large peak was considered to represent the transformant. The average area for the seeds constituting the small peak (a pixel number of 1,586) was found to be substantially consistent with that for the wild-type seeds (a pixel number of 1,591), and the average area for the seeds constituting the large peak (a pixel number of 2,429) was found to be greater than that for the wild-type seeds by approximately 50%.

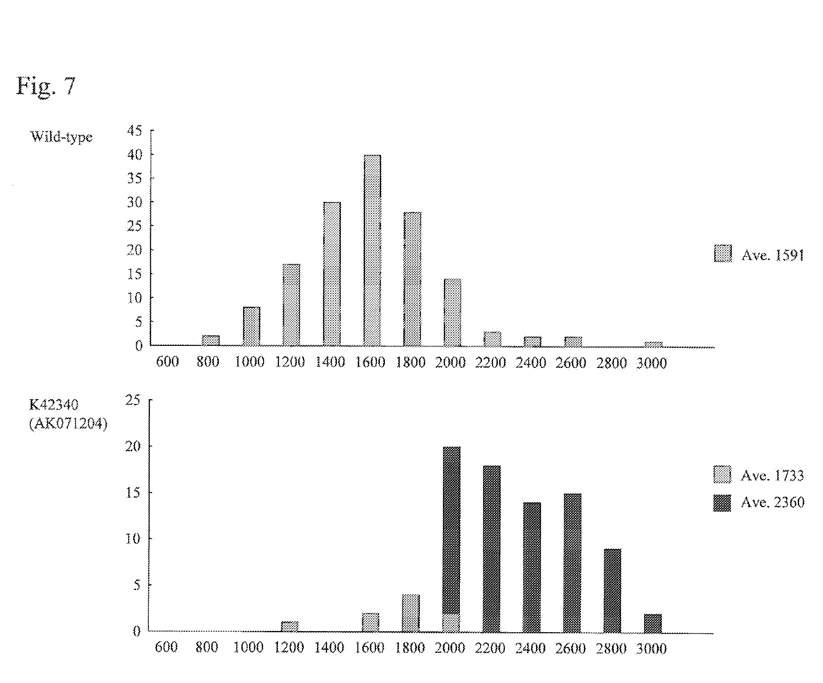

Fig. 7 shows histograms showing the areas of wild-type seeds and T2 generation seeds of K42340 (i.e., distribution of seed areas of K42340-overexpressing lines). The vertical axis indicates the seed number and the horizontal axis indicates the areas (i.e., the pixel numbers). K42340 exhibited an irregular peak. The peak of K42340 seeds was shifted to the right as compared with that of wild-type seeds. This indicates that seeds larger than wild-type seeds are dominant in this population.

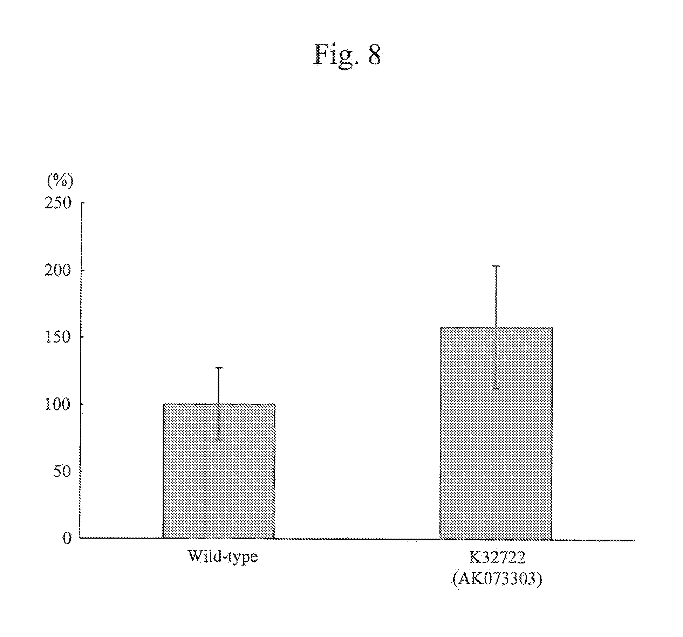

Fig. 8 shows the results of calculation of the volumes as spheroids of T2 generation seeds of K32722 as an example (i.e., the seed volumes of the K32722-overexpressing lines). Compared with wild-type seeds, the volumes of the K32722 seeds increased by approximately 50%.

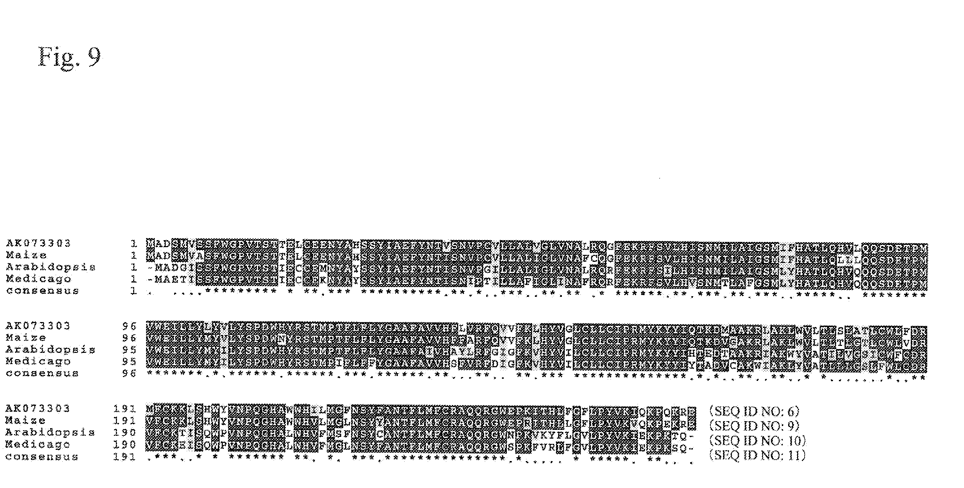

Fig. 9 shows the results of a comparison of the amino acid sequence (SEQ ID NO: 6) of DNA (AK073303) introduced into K32722, the amino acid sequence (SEQ ID NO: 9) of corn (family. *Gramineae;* genus: *Zea*), the amino acid sequence (SEQ ID NO: 10) of *Arabidopsis thaliana* (family: *Brassicaceae;* genus: *Arabidopsis*), and the amino acid sequence (SEQ ID NO: 11) of alfalfa (family: *Leguminosae;* genus: *Medicago*). The results demonstrate that DNA of AK073303 is highly conserved across plants of different families. It is considered that introduction of DNAs encoding the amino acid sequences as shown in SEQ ID NO: 9, SEQ ID NO: 10, and SEQ ID NO: 11 into plants results in increased seed size.

All publications, patents, and patent applications cited herein are incorporated herein by reference in their entirety.

## Claims

1. A transgenic plant into which DNA encoding any of proteins (a) to (c) below has been introduced so as to be capable of being expressed therein:
(a) a protein comprising the amino acid sequence as shown in SEQ ID NO: 2, 4, 6, or 8;
(b) a protein comprising an amino acid sequence derived from the amino acid sequence as shown in SEQ ID NO: 2, 4, 6, or 8 by deletion, substitution, or addition of one or several amino acids and having activity of increasing seed size; or
(c) a protein comprising an amino acid sequence having 90% or higher identity with the amino acid sequence as shown in SEQ ID NO: 2, 4, 6, or 8 and having activity of increasing seed size.

2. A transgenic plant into which any of DNAs (d) to (g) below has been introduced so as to be capable of being expressed therein:
(d) DNA comprising the nucleotide sequence as shown in SEQ ID NO: 1, 3, 5, or 7;
(e) DNA comprising a nucleotide sequence derived from the nucleotide sequence as shown in SEQ ID NO: 1, 3, 5, or 7 by deletion, substitution, or addition of one or several nucleotides and encoding a protein having activity of increasing seed size;
(f) DNA comprising a nucleotide sequence having 90% or higher identity with the nucleotide sequence as shown in SEQ ID NO: 1, 3, 5, or 7 and encoding a protein having activity of increasing seed size; or
(g) DNA hybridizing, under stringent conditions, to DNA comprising a nucleotide sequence complementary to DNA consisting of the nucleotide sequence as shown in SEQ ID NO: 1, 3, 5, or 7 and encoding a protein having activity of increasing seed size.

3. A transgenic plant into which DNA encoding any of proteins (h) to (j) below has been introduced so as to be capable of being expressed therein:
(h) a protein comprising the amino acid sequence as shown in SEQ ID NO: 9, 10, or 11;
(i) a protein comprising an amino acid sequence derived from the amino acid sequence as shown in SEQ ID NO: 9, 10, or 11 by deletion, substitution, or addition of one or several amino acids and having activity of increasing seed size; or
(j) a protein comprising an amino acid sequence having 90% or higher identity with the amino acid sequence as shown in SEQ ID NO: 9, 10, or 11 and having activity of increasing seed size.

4. The transgenic plant according to any one of claims 1 to 3, wherein the activity of increasing seed size is activity of increasing surface area, volume, or weight of seed.

5. The transgenic plant according to any one of claims 1 to 4, which is a plant, part of a plant, cultured plant cell, or seed.

6. A recombinant vector comprising DNA encoding any of proteins (a) to (c) below:
(a) a protein comprising the amino acid sequence as shown in SEQ ID NO: 2, 4, 6, or 8;
(b) a protein comprising an amino acid sequence derived from the amino acid sequence as shown in SEQ ID NO: 2, 4, 6, or 8 by deletion, substitution, or addition of one or several amino acids and having activity of increasing seed size; or
(c) a protein comprising an amino acid sequence having 90% or higher identity with the amino acid sequence as shown in SEQ ID NO: 2, 4, 6, or 8 and having activity of increasing seed size.

7. A recombinant vector comprising any of DNAs (d) to (g) below:
(d) DNA comprising the nucleotide sequence as shown in SEQ ID NO: 1, 3, 3, or 7;
(e) DNA comprising a nucleotide sequence derived from the nucleotide sequence as shown in SEQ ID NO: 1, 3, 5, or 7 by deletion, substitution, or addition of one or several nucleotides and encoding a protein having activity of increasing seed size;
(f) DNA comprising a nucleotide sequence having 90% or higher identity with the nucleotide sequence as shown in SEQ ID NO: 1, 3, 5, or 7 and encoding a protein having activity of increasing seed size; or
(g) DNA hybridizing, under stringent conditions, to DNA comprising a nucleotide sequence complementary to DNA consisting of the nucleotide sequence as shown in SEQ ID NO: 1, 3, 5, or 7 and encoding a protein having activity of increasing seed size.

8. A recombinant vector comprising DNA encoding any of proteins (h) to (j) below: (h) a protein comprising the amino acid sequence as shown in SEQ ID NO: 9, 10, or 11;
(i) a protein comprising an amino acid sequence derived from the amino acid sequence as shown in SEQ ID NO: 9, 10, or 11 by deletion, substitution, or addition of one or several amino acids and having activity of increasing seed size; or
(j) a protein comprising an amino acid sequence having 90% or higher identity with the amino acid sequence as shown in SEQ ID NO: 9, 10, or 11 and having activity of increasing seed size.

9. A method for producing a transgenic plant comprising introducing DNA encoding any of proteins (a) to (c) below into a plant cell and cultivating a plant:
(a) a protein comprising the amino acid sequence as shown in SEQ ID NO: 2, 4, 6, or 8;
(b) a protein comprising an amino acid sequence derived from the amino acid sequence as shown in SEQ ID NO: 2, 4, 6, or 8 by deletion, substitution, or addition of one or several amino acids and having activity of increasing seed size; or
(c) a protein comprising an amino acid sequence having 90% or higher identity with the amino acid sequence as shown in SEQ ID NO: 2, 4, 6, or 8 and having activity of increasing seed size.

10. A method for producing a transgenic plant comprising introducing any of DNAs (d) to (g) below into a plant cell and cultivating a plant:
(d) DNA comprising the nucleotide sequence as shown in SEQ ID NO: 1, 3, 5, or 7;
(e) DNA comprising a nucleotide sequence derived from the nucleotide sequence as shown in SEQ ID NO: 1, 3, 5, or 7 by deletion, substitution, or addition of one or several nucleotides and encoding a protein having activity of increasing seed size;
(f) DNA comprising a nucleotide sequence having 90% or higher identity with the nucleotide sequence as shown in SEQ ID NO: 1, 3, 5, or 7 and encoding a protein having activity of increasing seed size; or
(g) DNA hybridizing, under stringent conditions, to DNA comprising a nucleotide sequence complementary to DNA consisting of the nucleotide sequence as shown in SEQ ID NO: 1, 3, 5, or 7 and encoding a protein having activity of increasing seed size.

11. A method for producing a transgenic plant comprising introducing DNA encoding any of proteins (h) to (j) below into a plant cell and cultivating a plant:
(h) a protein comprising the amino acid sequence as shown in SEQ ID NO: 9, 10, or 11;
(i) a protein comprising an amino acid sequence derived from the amino acid sequence as shown in SEQ ID NO: 9, 10, or 11 by deletion, substitution, or addition of one or several amino acids and having activity of increasing seed size; or
(j) a protein comprising an amino acid sequence having 90% or higher identity with the amino acid sequence as shown in SEQ ID NO: 9, 10, or 11 and having activity of increasing seed size.

12. The method according to any one of claims 9 to 11, wherein DNA is introduced with the use of the recombinant vector according to any of claims 6 to 8.
